# FASCICULE DE BREVET EUROPEEN

(11) **EP 0 430 771 B1**
(45) Date de publication et mention de la délivrance du brevet: **20.07.1994**
(21) Numéro de dépôt: 90403300.8
(22) Date de dépôt: 22.11.1990
(51) Int. Cl.: C07D 209/44, A61K 31/40

(54) **Dérivés de l'isoindolone, leur préparation et leur utilisation comme intermédiaires pour la préparation d'antagonistes de la substance P**
Isoindolonderivate, ihre Herstellung und ihre Verwendung als Zwischenprodukte zur Herstellung von Substanz-P-Antagonisten
Isoindolone derivatives, their preparation and their use as intermediates for the preparation of substance P antagonists

(30) Priorité: 23.11.1989 FR 8915407
(43) Date de publication de la demande: 05.06.1991
(73) Titulaire: RHONE-POULENC SANTE, 92165 Antony Cédex (FR)
(72) Inventeur: Dubroeucq, Marie-Christine, F-95880 Enghien Les Bains (FR); Moutonnier, Claude, F-92350 Le Plessis Robinson (FR); Peyronel, Jean-François, F-91120 Palaiseau (FR); Tabart, Michel, F-75013 Paris (FR); Truchon, Alain, F-69004 Lyon (FR)
(74) Mandataire: Savina, Jacques

(56) Documents cités:
- US-A- 2 858 314
- US-A- 4 042 707

## Description

La présente invention concerne des dérivés de l'isoindolone de formule générale : 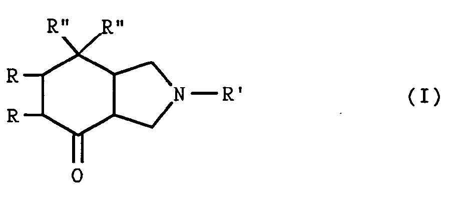 dans laquelle les radicaux R représentent des atomes d'hydrogène ou forment ensemble une liaison, le symbole R' représente un atome d'hydrogène ou un radical facilement éliminable défini ci-après et les symboles R" sont identiques et représentent des radicaux phényle pouvant être substitués par un atome d'halogène ou un radical méthyle en position ortho ou méta, ainsi que leurs sels.

Le brevet américain 4 042 707 a décrit antérieurement les dérivés de l'isoindole de formule générale : 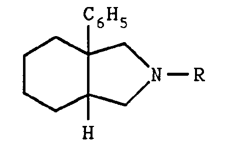 utiles dans le domaine pharmaceutique.

Les dérivés de l'isoindolone selon la présente invention, sont particulièrement intéressants comme intermédiaires pour la préparation de produits antagonistes de la substance P.

Dans la formule générale (I) lorsque R' représente un groupement facilement éliminable, ce radical peut être un radical allyle ou un radical choisi parmi les groupements de structure : 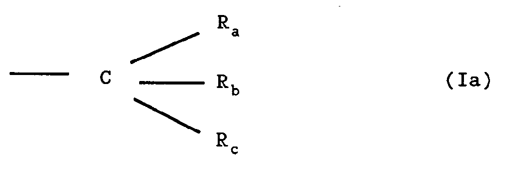 dans laquelle Rₐ et R_{b} sont des atomes d'hydrogène ou des radicaux phényle éventuellement substitués (par un atome d'halogène, un radical alcoyle, alcoyloxy ou nitro), et Rₑ est défini comme Rₐ et R_{b} ou représente un radical alcoyle ou alcoyloxyalcoyle, l'un au moins de Rₐ, R_{b} et Rₑ étant un radical phényle substitué ou non. Lorsque les radicaux définis par R" portent des substituants halogène, ces derniers sont avantageusement choisis parmi le fluor ou le chlore.

De plus, i il est entendu que les radicaux alcoyle cités ci-dessus ou qui sont cités ci-après sont droits ou ramifiés et contiennent sauf mention spéciale 1 à 4 atomes de carbone.

Les produits de formule générale (I) présentent des formes stéréoisomères, il est entendu que les dérivés de l'isoindolone de forme cis (3aR,7aR) à l'état pur, ou sous forme de mélange des formes cis (3aRS,7aRS), entrent dans le cadre de la présente invention.

Selon l'invention le dérivé de l'isoindolone de formule générale (I) peut être obtenu par réaction de cycloaddition par action d'un dérivé silylé de formule générale : 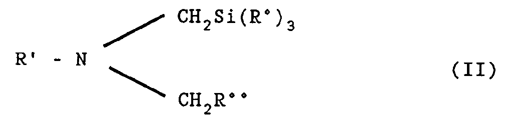 dans laquelle R' est le radical facilement éliminable défini précédemment, (R°)₃ représente des radicaux alcoyle ou des radicaux alcoyle et phényle et R°° représente un radical alcoyloxy, cyano ou phénylthio, sur un dérivé de la cyclohexènone de formule générale : 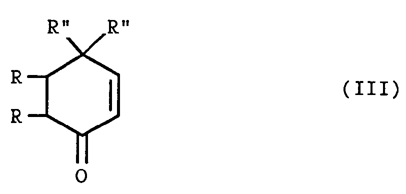 dans laquelle R et R" sont définis comme précédemment, suivie éventuellement de l'élimination du radical R' facilement éliminable, lorsque l'on veut obtenir une isoindolone de formule générale (I) pour laquelle R' est un atome d'hydrogène.

La réaction de cycloaddition s'effectue en présence d'une quantité catalytique d'un acide dans un solvant organique tel qu'un solvant chloré (dichlorométhane, dichloréthane par exemple), dans un hydrocarbure aromatique, dans un nitrile (acétonitrile) ou dans un éther, à une température comprise entre 0°C et la température de reflux du mélange réactionnel.

Les acides utilisés sont avantageusement choisis parmi l'acide trifluoroacétique, l'acide acétique, l'acide méthanesulfonique ou les acides cités dans les références mentionnées ci-après pour la préparation des dérivés silylés de formule générale (II).

L'élimination du radical R' facilement éliminable lorsque l'on veut obtenir une isoindolone pour laquelle R' est hydrogène s'effectue par toute méthode connue qui n'affecte pas le reste de la molécule.

Notamment, lorsque R est un atome d'hydrogène, et lorsque R' est autre qu'un radical allyle, le groupement R' peut être éliminé par hydrogénation catalytique en présence de palladium. Généralement, la réaction s'effectue en milieu acide, dans un solvant tel qu'un alcool (méthanol, éthanol), dans l'eau ou directement dans l'acide acétique ou l'acide formique, à une température comprise entre 20 et 60°C.

Lorsque R' est un radical benzhydryle ou trityle, l'élimination peut être effectuée par traitement en milieu acide, en opérant à une température comprise entre 0°C et la température de reflux du mélange réactionnel, dans un alcool, dans un éther, dans l'eau ou directement dans l'acide acétique, l'acide formique ou l'acide trifluoroacétique. Le groupement R' peut être également éliminé en faisant agir le chloroformiate de vinyle, le chloroformiate de chloro-1 éthyle ou le chloroformiate de phényle, en passant intermédiairement par un produit de formule générale : 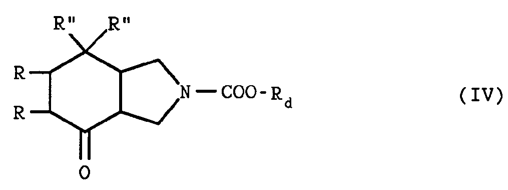 dans laquelle R et R" sont définis comme précédemment, et R_{d} est un radical vinyle, chloro-1 éthyle ou phényle, puis par élimination du radical COO R_{d} par traitement acide.

L'action du chloroformiate s'effectue dans un solvant organique tel qu'un solvant chloré (dichlorométhane, dichloréthane, chloroforme par exemple), un éther (tétrahydrofuranne, dioxanne par exemple), ou une cétone (acétone par exemple) ou dans un mélange de ces solvants, à une température comprise entre 20°C et la température de reflux du mlange réactionnel. L'élimination du radical COO R_{d} est effectuée par traitement en milieu acide par exemple par l'acide trifluoroacétique, formique, méthanesulfonique, p.tolune sulfonique, chlorhydrique ou bromhydrique dans un solvant tel qu'un alcool, un éther, un ester, un nitrile, un mélange de ces solvants ou dans l'eau, à une température comprise entre 0 °C et la température de reflux du mélange réactionnel. Dans les conditions d'élimination des radicaux R' citées précédemment, le dérivé de l'isoindolone de formule générale (I) est obtenu à l'état de sel de l'acide employé. Le produit peut être libéré de son sel par les méthodes habituelles.

Le dérivé silylé de formule générale (II) peut être obtenu selon les méthodes décrites par :
- Y Terao et coll., Chem. Pharm. Bull., 33, 2762 (1985);
- A. Hosomi et coll., Chem. Lett., 1117 (1984) ;
- A. Padwa et coll., Chem. Ber., 119, 813 (1986) ou
- Tetrahedron, 41, 3529 (1985).

Le dérivé de la cyclohexènone de formule générale (III) peut être préparé comme décrit ci-après dans les exemples.

Selon l'invention, les dérivés de l'isoindolone de formule générale (I) peuvent également être préparés par action d'une oxazolidinone de formule générale : 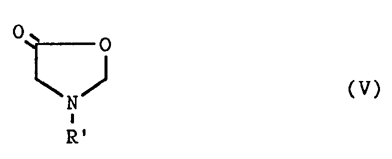 dans laquelle R' est un groupement facilement éliminable tel que défini précédemment, sur une cyclohexènone de formule générale (III), suivie le cas échéant de l'élimination du radical facilement éliminable R', lorsque l'on veut obtenir un dérivé de l'isoindolone pour lequel R' est un atome d'hydrogène.

La réaction s'effectue par chauffage à une température comprise entre 80°C et la température de reflux du mélange réactionnel, dans un solvant tel qu'un hydrocarbure aromatique (toluène ou xylène par exemple), un éther (dioxanne, glymes) ou un solvant halogéné (trichloréthane, chlorobenzène par exemple).

Le cas échéant, l'élimination du radical R' s'effectue comme décrit précédemment.

Les oxazolidinones de formule générale (V) peuvent être préparées selon ou par analogie avec la méthode décrite par M. Joucla et coll., Bull. Soc. Chim. Fr., 579 (1988).

Selon l'invention, les dérivés de l'isoindolone de formule générale (I) pour lesquels R est un atome d'hydrogène et R' est défini comme précédemment à l'exception de pouvoir représenter un radical trityle, peuvent également être obtenus par réaction de Mannich à partir d'un dérivé de formule générale : 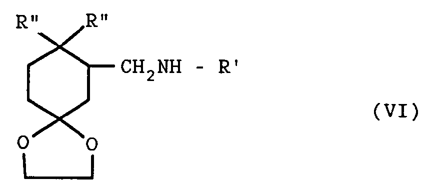 dans laquelle R' et R" sont définis comme ci-dessus.

La réaction s'effectue en mileu acide, en présence de formaldéhyde à une température comprise entre 20°C et la température de reflux du mélange réactionnel, dans un solvant tel qu'un alcool (méthanol, éthanol, iso- propanol, polyéthylèneglycol par exemple) ou un éther (dioxanne, tétrahydrofuranne, glyme par exemple). On opère avantageusement en présence d'un acide minéral ou organique comme l'acide sulfurique, chlorhydrique, méthane sulfonique ou p.toluènesulfonique.

Lorsque R' est autre que l'atome d'hydrogène, le dérivé aminé de formule générale (VI) peut être obtenu à partir du dérivé pour lequel R' est un atome d'hydrogène par toute méthode connue pour la mise en place d'un radical protecteur d'amino, qui n'altère pas le reste de la molécule.

On opère notamment selon les méthodes décrites par T.W. Greene, Protective Groups in Organic Synthesis, A. Wiley, Interscience Publication (1981) ou par Mc Omie, Protective Groups in Organic Chemistry, Plenum Press (1973).

Lorsque R' est un radical benzyle ou benzyle substitué, il peut être avantageux de préparer un amide de formule générale : 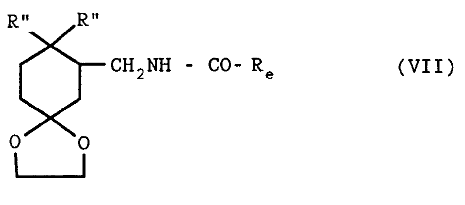 dans laquelle Rₑ est un radical phényle ou phényle substitué, par action du chlorure de l'acide correspondant, sur l'amine de formule générale (VI) dans laquelle R' est un atome d'hydrogène, puis de réduire l'amide obtenu par l'hydrure d'aluminium et de lithium en milieu anhydre.

La préparation de l'amide s'effectue par exemple en présence d'une base azotée comme la triéthylamine, dans un solvant organique anhydre (dichlorométhane par exemple) à une température comprise entre -20 et 40°C. La réduction s'effectue dans un solvant organique tel qu'un éther (tétrahydrofuranne par exemple) à une température comprise entre 0°C et la température de reflux du mélange réactionnel.

L'amine de formule générale (VI) pour laquelle R' est un atome d'hydrogène, peut être préparée à partir de la cyclohexénone de formule générale (III) comme décrit ci-après dans les exemples.

Selon l'invention, les dérivés de l'isoindolone de formule générale (I) dans laquelle R et R' sont des atomes d'hydrogène, peuvent également être obtenus par hydrogénation catalytique d'une formyl-2 nitrométhyl-3 cyclohexanone de formule générale : 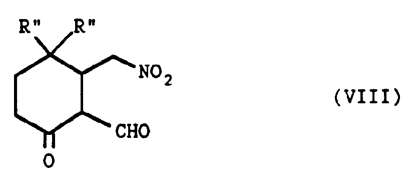 dans laquelle R" est défini comme précédemment.

La réaction s'effectue en milieu acide, en présence de palladium.

Il est avantageux d'opérer sous pression dans l'acide acétique à une température comprise entre 20 et 80°C. Le formyl-2 nitrométhyl-3 cyclohexanone de formule (VIII) peut être obtenue à partir de la diphényl-4,4 cyclohexanone comme décrit ci-après dans les exemples.

Selon l'invention, lorsque l'on veut obtenir un produit de formule générale (I) de forme (3aR,7aR), la séparation s'effectue selon les méthodes connues et compatibles avec la molécule. A titre d'exemple, la séparation des isomères du dérivé de l'isoindolone de formule générale (I) pour lequel R' est un atome d'hydrogène peut être effectuée par formation d'un sel au moyen d'un acide optiquement actif (notamment l'acide L(+) ou D(-) mandélique ou l'acide dibenzoyltartrique), puis séparation des isomères par cristallisation. L'isomère recherché est libéré de son sel par traitement en milieu basique.

Les nouveaux produits de formule générale (I) ainsi que leurs sels sont utiles comme intermédiaires pour la préparation de dérivés de l'isoindolone qui antagonisent les effets de la substance P et répondent à la formule générale : 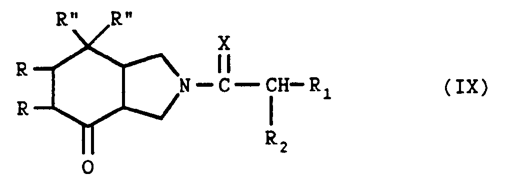 dans laquelle
- les radicaux Asont identiques et représentent des atomes d'hydrogène ou forment ensemble une liaison,
- les radicaux R" sont définis comme précédemment,
- le symbole X représente un atome d'oxygène, de soufre ou un radical N-R₃ pour lequel R₃ est un atome d'hydrogène, un radical alcoyle contenant 1 à 12 atomes de carbone, éventuellement substitué [par un ou plusieurs radicaux carboxy, dialcoylamino, acylamino, carbamoyle, alcoylcarbamoyle, dialcoylcarbamoyle, alcoyloxycarbonyle (les portions alcoyle de ces radicaux pouvant porter un substituant dialcoylamino ou phényle), ou par des radicaux phényle, phényle substitué par (halogène, alcoyle, alcoyloxy ou dialcoylamino), naphtyle, thiényle, furyle, pyridyle ou imidazolyle] ou un radical dialcoylamino,
- le symbole R₁ représente un radical phényle éventuellement substitué par un ou plusieurs atomes d'halogène ou radicaux hydroxy, alcoyle pouvant être éventuellement substitués (par des atomes d'halogène ou des radicaux amino, alcoylamino ou dialcoylamino), alcoyloxy ou alcoylthio pouvant être éventuellement substitués (par des radicaux hydroxy ou dialcoylamino dont les parties alcoyle peuvent former avec l'atome d'azote auquel elles sont rattachées, un hétérocycle à 5 à 6 chaînons pouvant contenir un autre hétéroatome choisi parmi l'oxygène, le soufre ou l'azote éventuellement substitué par un radical alcoyle), ou substitué par des radicaux amino, alcoylamino, dialcoylamino dont les parties alcoyle peuvent former avec l'atome d'azote auquel elles sont rattachées, un hétérocycle tel que défini ci-dessus, ou représente un radical cyclohexadiènyle, naphtyle ou hétérocyclyle mono ou polycyclique, saturé ou insaturé contenant 5 à 9 atomes de carbone et un ou plusieurs hétéroatomes choisis parmi l'oxygène, l'azote ou le soufre et
- le symbole R₂ représente un atome d'hydrogène ou d'halogène ou un radical hydroxy, alcoyle, aminoal- coyle, alcoylaminoalcoyle, dialcoylaminoalcoyle, alcoyloxy, alcoylthio, alcyloxy, carboxy, alcoyloxycarbonyle, dialcoylaminoalcoyloxycarbonyle, benzyloxycarbonyle, amino, acylamino ou alcoyloxycarbony- lamino.

Dans la formule générale (IX) les radicaux alcoyle ou acyle contiennent 1 à 4 atomes de carbone en chaîne droite ou ramifiée ; lorsque R₁ ou R₃ contient un atome d'halogène, ce dernier est choisi parmi le chlore, le brome, le fluor ou l'iode ;

lorsque R₁ représente un radical hétérocyclyle mono ou polycyclique, saturé ou insaturé, à titre d'exemple il peut être choisi parmi thiényle, furyle, pyridyle, dithiinyle, indolyle, isoindolyle, thiazolyle, isothiazolyle, oxa- zolyle, imidazolyle, pyrrolyle, triazolyle, thiadiazolyle, quinolyle, isoquinolyle, ou naphtyridinyle.

Les dérivés de l'isoindolone de formule générale (IX) peuvent être obtenus par action de l'acide de formule générale : 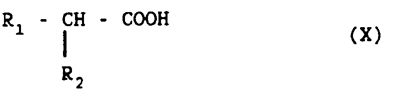 ou d'un dérivé réactif de cet acide, dans lequel R₁ et R₂ sont définis comme précédemment, sur un dérivé de l'isoindole de formule générale (I) dans laquelle R' est un atome d'hydrogène et R et R" sont définis comme précédemment, suivie le cas échéant de la transformation de l'amide obtenu en thioamide ou en une amidine pour laquelle X représente un radical N-R₃, R₃ ayant la définition donnée précédemment.

Il est entendu que, les radicaux amino, alcoylamino ou carboxy contenus dans R₁ et/ou R₂ sont de préférence préalablement protégés. La protection s'effectue partout groupement compatible, dont la mise en place et l'élimination n'affectent pas le reste de la molécule.

Notamment, on opère selon les méthodes décrites parT.W. Greene, Protective- Groups in Organic Synthesis, AWiley - Interscience Publication (1981), ou par Mc Omie, Protective Groups in Organic Chemistry, Plenum Press (1973).

A titre d'exemple,
- les groupements amino ou alcoylamino peuvent être protégés par des radicaux méthoxycarbonyle, éthoxycarbonyle, t.butoxycarbonyle, allyloxycarbonyle, vinyloxycarbonyle, trichloréthoxycarbonyle, tri- chloracétyle, trifluoracétyle, chloracétyle, trityle, benzhydryle, benzyle, allyle, formyle, acétyle, benzyloxycarbonyle ou ses dérivés substitués ;
- les groupements acides peuvent être protégés par des radicaux méthyle, éthyle, t.butyle, benzyle, benzyle substitué ou benzhydryle.

De plus, lorsque R₂ représente un radical hydroxy, il est préférable de protéger préalablement ce radical. La protection s'effectue par exemple par un radical acétoxy, trialcoylsilyle, benzyle, ou sous forme d'un carbonate.

Lorsque l'on effectue la condensation du produit de formule générale (X) sous sa forme acide (dont le cas échéant les substituants amino, alkylamino, carboxy et/ou hydroxy sont préalablement protégés), on opère généralement en présence d'un agent de condensation tel qu'un carbodiimide (par exemple dicyclohexylcar- bodiimide), le NN'-carbonyldiimidazole ou l'éthoxy-2 éthoxycarbonyl-1 dihydro-1,2 quinoléine, dans un solvant organique tel qu'un solvant chloré (dichlorométhane, dichloréthane, chloroforme par exemple), un éther (tétrahydrofuranne, dioxanne par exemple), un ester (acétate d'éthyle par exemple), un amide (diméthylacéta- mide, diméthylformamide par exemple), un nitrile (acétonitrile par exemple), une cétone (acétone par exemple) ou dans un hydrocarbure aromatique comme le toluène par exemple, à une température comprise entre -20 et 40°C, puis on transforme le cas échéant le produit obtenu en un thioamide ou une amidine, et élimine le cas échéant les radicaux protecteurs.

Lorsque l'on effectue la condensation d'un dérivé réactif de l'acide de formule générale (X), on opère avantageusement au moyen du chlorure d'acide, de l'anhydride, d'un anhydride mixte ou d'un ester réactif dans lequel le reste de l'ester est un radical succinimido, benzotriazolyl-1, nitro-4 phényle, dinitro-2,4 phényle, pen- tachlorophényle ou phtalimido. La réaction s'effectue généralement à une température comprise entre -40 et +40_{°}C, dans un solvant chloré, un éther, un amide, une cétone, ou un mélange de ces solvants, en présence d'un accepteur d'acide comme une base organique azotée, un époxyde ou un carbodiimide, ou bien en milieu hydroorganique en présence d'un agent alcalin de condensation, puis on transforme le cas échéant l'amide obtenu en un thioamide et/ou en une amidine telle que définie précédemment.

La transformation de l'amide de formule générale (IX) en un thioamide s'effectue par toute méthode de thio- nation qui n'altère pas le reste de la molécule.

On opère notamment par action du réactif de Lawesson [bis (méthoxy-4 phényl)-2,4 dithioxo-2,4 dithiadiphos- phétane-1,3,2,4], ou par action du pentasulfure de phosphore, dans un solvant organique tel qu'un éther (tétrahydrofuranne, diméthoxy-1,2 éthane, dioxanne par exemple), un hydrocarbure aromatique (toluène par exemple), à une température comprise entre 0°C et la température de reflux du mélange réactionnel.

La transformation de l'amide de formule générale (IX) en une amidine pour laquelle X est un radical N-R₃ s'effectue, soit directement, soit par l'intermédiaire du thioamide correspondant, en préparant le dérivé de l'isoindolium de formule générale : 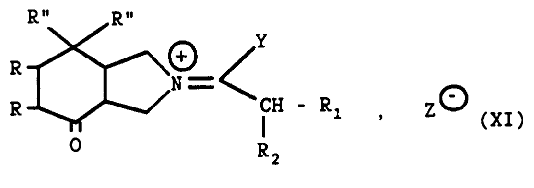 dans laquelle R, R", R₁ et R₂ sont définis comme précédemment et soit Y représente un atome de chlore, un radical méthoxy ou éthoxy et Z- représente un ion chlorure, tétrafluoroborate, fluorosulfonate, trifluorométhyl- sulfonate, méthylsulfate, ou éthylsulfate soit Y représente un atome de chlore ou un radical méthylthio, éthyl- thio, benzylthio ou alcoyloxycarbonylméthylthio et Z- est défini comme ci-dessus ou représente un ion iodure ou bromure, puis en faisant agir une amine de formule générale : 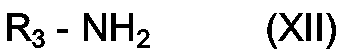 dans laquelle R₃ est défini comme précédemment.

La préparation du dérivé de l'isoindolium de formule générale (XI) dans laquelle Y est un atome de chlore ou un radical méthoxy ou éthoxy s'effectue par action d'un réactif tel que le phosgène, l'oxychlorure de phosphore, le pentachlorure de phosphore, le chlorure de thionyle, le chlorure d'oxalyle, le chloroformate de tri- chlorométhyle, le tétrafluoroborate de triéthyl (ou de triméthyl) oxonium, le triflate de méthyl (ou d'éthyle), le fluorosulfonate de méthyle (ou d'éthyle) ou le sulfate de méthyle (ou d'éthyle). La préparation du dérivé de l'isoindolium de formule générale (XI) dans laquelle Y est un atome de chlore, un radical méthyl (ou éthyl) thio, benzylthio, ou alcoyloxycarbonylméthylthio s'effectue à partir du dérivé de l'isoindolone de formule générale (IX) dans laquelle X est un atome de soufre, par action d'un réactif tel que cité précédemment ou par action du bromure ou de l'iodure de méthyle, d'éthyle ou de benzyle. La réaction s'effectue dans un solvant chloré (dichlorométhane, dichloréthane par exemple) ou dans un hydrocarbure aromatique (toluène par exemple), à une température comprise entre 0°C et la température de ref lux du mélange réactionnel. Lorsque l'on opère à partir du thioamide de formule générale (IX), il est également possible d'utiliser des solvants tels que les éthers, les cétones, les esters ou les nitriles. L'action de l'amine de formule générale (XII) sur le dérivé de formule générale (XI) s'effectue dans un solvant organique anhydre tel qu'un solvant chloré (dichlorométhane, dichloréthane parexemple), dans un mélange alcool-solvant chloré, dans un éther (tétrahydrofurane par exemple), dans un ester (par exemple acétate d'éthyle), dans un solvant aromatique (toluène par exemple) ou dans un mélange de ces solvants, à une température comprise entre -20°C et la température de reflux du mélange réactionnel.

Il n'est pas indispensable d'avoir isolé le dérivé de l'isoindolium de formule générale (XI) pour le mettre en oeuvre dans cette réaction.

Les acides de formule générale (X) peuvent être préparés selon les méthodes décrites ci-après dans les exemples, ou par analogie avec ces méthodes.

Les nouveaux dérivés de l'isoindolone de formule générale (I) ainsi que les dérivés de formule générale (IX) peuvent être purifiés le cas échéant par des méthodes physiques telles que la cristallisation ou la chromatographie. Le cas échéant les nouveaux produits selon l'invention peuvent être transformés en sels d'addition avec les acides. Comme exemples peuvent être cités les sels formés avec les acides minéraux (chlorhydrates, bromhydrates, sulfates, nitrates, phosphates, tétrafluoroborates, fluorosulfonates) ou avec les acides organiques (succinates, fumarates, tartrates, acétates, propionates, maléates, citrates, méthanesulfonates, p.toluè- nesulfonates, trifluorométhylsulfonates, méthylsulfates, éthylsulfates, iséthionates, ou avec des dérivés de substitution de ces composés).

Les dérivés de l'isoindolone de formule générale (IX) antagonisent les effets de la substance P et sont de ce fait particulièrement intéressants dans les domaines de l'analgésie, de l'inflammation, de l'asthme, des allergies, sur le système nerveux central, sur le système cardiovasculaire, comme antispasmodique ou sur le système immunitaire, ainsi que dans la stimulation des secrétions lacrymales. Leur activité a été mise en évidence à des doses comprises entre 5 et 2000 nM dans la technique décrite par C.M. Lee et coll., Mol. Pharmacol., 23, 563-69 (1983).

D'un interêt particulier sont les produits de formule générale (I) pour lesquels les radicaux R sont des atomes d'hydrogène ou forment ensemble une liaison, le symbole R' est un atome d'hydrogène ou un radical benzyle et les symboles R" sont des radicaux phényle éventuellement substitués en position ortho ou méta par des atomes de fluor ou de chlore, ou par un radical méthyle.

Les produits suivants se sont montrés spécialement intéressants: la diphényl-7,7 perhydroisoindolone-4 sous ses formes (3aR,7aR) ou (3aRS,7aRS), ainsi que ses sels d'addition avec les acides;
la bis(fluoro-3 phényl)-7,7 perhydroisoindolone-4 sous ses formes (3aR,7aR) ou (3aRS,7aRS), ainsi que ses sels d'addition acides;
la bis(fluoro-2 phényl)-7,7 perhydroisoindolone-4 sous ses formes (3aR,7aR) ou (3aRS,7aRS), ainsi que ses sels d'addition avec les acides;
la bis(chloro-3 phényl)-7,7 perhydroisoindolone-4 sous ses formes (3aR,7aR) ou (3aRS,7aRS), ainsi que ses sels d'addition acides;
la bis(tolyl-3)-7,7 perhydroisoindolone-4 sous ses formes (3aR,7aR) ou (3aRS,7aRS), ainsi que ses sels d'addition avec les acides.

Les exemples suivants, donnés à titre non limitatif, illustrent la présente invention. Dans les exemples qui suivent, il est entendu que, sauf mention spéciale, les spectres de RMN du proton ont été faits à 250 MHz dans le diméthylsulfoxyde ; les déplacements chimiques sont exprimés en ppm.

### EXEMPLE 1

A une solution de 155 g de diphényl-4,4 cyclohexène-2 one-1 et de 202 cm³ de N-butoxyméthyl N-triméthylsilylméthyl benzylamine dans 1000 cm³ de dichlorométhane sec, on ajoute 5 gouttes d'acide trifluoroacétique et chauffe le mélange réactionnel au reflux pendant 45 minutes. On ajoute 50 cm³ de N-butoxyméthyl N-triméthylsilylméthyl benzylamine et 3 gouttes d'acide trifluoroacétique et agite encore 45 minutes au reflux avant d'ajouter de nouveau 25 cm³ de N-butoxyméthyl N-tri méthylsilylméthyl benzylamine et 3 gouttes d'acide trifluoro acétique. Le mélange réactionnel est agité au reflux 45 minutes puis traité par 50 g de carbonate de potassium, filtré et concentré à sec sous pression réduite (2,7 kPA). Le résidu est dissous dans 200 cm³ d'oxyde d'isopropyle et la solution refroidie à 0°C pendant 1 heure. Les cristaux sont essorés, lavés 2 fois par 15 cm³ d'oxyde d'isopropyle et séchés pour donner 193 g de benzyl-2 diphényl-7,7 perhydroisoindoline-4-(3aRS,7aRS) sous la forme de cristaux blancs fondant à 132°C.

La N-butoxyméthyl N-triméthylsilylméthyl benzylamine peut être préparée selon la méthode de Y. Terao et coll., Chem. Pharm. Bull., 33, 2762 (1985).

### EXEMPLE 2

A 15 g de palladium sur charbon à 10%, on ajoute 150 g de benzyl-2 diphényl-7,7 perhydroisoindolone-4 (3aRS,7aRS), 1500 cm³ de méthanol et 450 cm³ d'acide chlorhydrique 1 N ; le mélange réactionnel est hydrogéné, sous agitation, à température ambiante et sous pression atmosphérique. Après 5 heures de réaction le volume théorique d'hydrogène a été absorbé ; le mélange réactionnel est filtré, puis concentré à sec sous pression réduite (2,7 kPa); le résidu est cristallisé dans 200 cm³ d'éthanol ; les cristaux obtenus sont essorés, lavés par 50 cm³ d'éthanol et séchés. On obtient 110 g de chlorhydrate de diphényl-7,7 perhydroisoindoline-4-(3aRS,7aRS) fondant à 270°C avec décomposition. Spectre de RMN de proton :
2,03 (Mt, 1 H du H en 5 ou 6) ; 2,3 (Mt, 1 H, 1 H du - H en 5 ou 6) ; 2,48 (DD, en partie masqué, 1 H du -CH₂- en 1 ) ; 2,69 (DD, 1 H, 1 H du -CH₂- en 1). 2.8 (Mt, 2H, -CH₂- en 6 ou 5) ; 3,34 (DD, en partie masqué , 1 H du -CH₂- en 3) ; 3,5 (Mt, 1 H, -CH- en 3a) ; 3,82 (DD, 1 H, 1 H du -CH₂- en 3) ; 3,95 (Mt, 1 H, -C^{H-} en 7a) ; 7,15 à 7,65 (Mt, 10H, aromatiques) ; 9,43 (Mf, 2H, -NH₂-Cl).
Spectre infra-rouge (KBr) bandes caractéristiques en cm-¹ :
3600-3300, 3100-3000, 3000-2850, 3100-2400, 1715, 1595, 1580, 1495, 1445, 1470, 775, 750, 705.

### EXEMPLE 3

A une solution de 193 g de benzyl-2 diphényl-7,7 perhydroisoindolone-4(3aRS,7aRS) dans 1225 cm³ de dichloro-1,2 éthane refroidie à +5_{°}C , on ajoute goutte à goutte en 10 minutes 56 cm³ de chloroformiate de vinyle. Après agitation 30 minutes entre 10°et 20°C le mélange réactionnel est chauffé au reflux pendant 90 minutes, refroidi et concentré à sec sous pression réduite (2,7 kPa puis 1 kPa). La masse cristalline obtenue est battue avec 200 cm³ d'oxyde d'isopropyle froid. Les cristaux obtenus sont essorés, lavés 2 fois par 100 cm³ d'oxyde d'isopropyle et séchés. On obtient 177 g de diphényl-7,7 vinyloxycarbonyl-2 perhydroisoindolone-4-(3aAS,7aAS) fondant à 178°C.

On traite 177 g de diphényl-7,7 vinyloxycarbonyl-2 perhydroisoindolone-4-(3aRS,7aRS) par 1000 cm³ d'une solution 5,7 N d'acide chlorhydrique dans le dioxanne sec pendant 30 minutes à 20°C. La solution est concentrée à sec sous pression réduite (2,7 kPa) et le résidu repris dans 500 cm³ d'éthanol et le mélange réactionnel est agité à 60°C pendant 30 minutes puis refroidi à + 5°C. Les cristaux obtenus sont essorés, lavés par 50 cm³ d'éthanol et séchés. On obtient 130 g de chlorhydrate de diphényl-7,7 perhydroisoindolone-4-(3aRS,7aRS) fondant à 270°C avec décomposition.

### EXEMPLE 4

Un mélange de 3,4 g de benzyl-2 diphényl-7,7 hexahydro-2,3,3a,4,7,7a 1 H-isoindolone-4 et de 0,92 cm³ de chloroformiate de vinyle dans 80 cm³ de dichloro-1,2 éthane est chauffé au reflux pendant 1 heure ; le mélange réactionnel est concentré à sec sous pression réduite (2,7kPa). Le résidu est cristallisé dans 20 cm³ d'éther éthylique. On obtient 2,6 g de diphényl-7,7 vinyloxycarbonyl-2 hexahydro-2,3,3a,4,7,7a 1 H-isoindolone-4, fondant à 162°C.

2,6 g de diphényl-7,7 vinyloxycarbonyl-2 hexahydro-2,3,3a,4,7,7a 1 H-isoindolone-4 sont agités dans 30 cm³ de dioxanne chlorhydrique 3N à température ambiante pendant 30 minutes ; le mélange est concentré à sec sous pression réduite (2,7 kPa). Le résidu est repris par 50 cm³ d'éthanol et chauffé au reflux pendant 30 minutes ; le mélange est concentré à sec sous pression réduite (2,7 kPa). Le résidu est cristallisé dans 20 cm³ d'éther éthylique les cristaux obtenus sont essorés et séchés. On obtient 2 g de chlorhydrate de diphényl-7,7 hexahydro-2,3,3a,4,7,7a 1 H-isoindolone-4, fondant à une température supérieure à 260°C.

La benzyl-2 diphényl-7,7 hexahydro-2,3,3a,4,7,7a 1 H-isoindolone-4-(3aRS,7aRS) peut être obtenue de la manière suivante :
A une solution de 7,7 g de diphényl-4,4 cyclohéxadiène-2,5 one-1 et de 11 cm³ de N-butoxyméthyl N-triméthylsilylméthyl benzylamine dans 80 cm³ de dichlorométhane sec, on ajoute 2 gouttes d'acide trifluoroacétique et chauffe le mélange réactionnel au reflux pendant 1 heure et demi. On ajoute 5 cm³ de N-butoxyméthyl N-triméthylsilylméthyl benzylamine supplémentaires ainsi que 2 gouttes d'acide trifluoroacétique et chauffe le mélange réactionnel pendant 1 heure et demi. Le mélange réactionnel est traité par 3 g de carbonate de potassium, filtré et concentré à sec sous pression réduite (2,7 kPa). Le résidu est cristallisé dans 15 cm³ d'oxyde d'isopropyle. Les cristaux obtenus sont essorés, lavés 5 cm³ d'oxyde d'isopropyle (2 fois) et séchés ; on obtient 4,4 g de benzyl-2 diphényl-7,7 hexahydro-2,3,3a,4,7,7a 1 H-isoindolone-4-(3aRS,7aRS), fondant à 132°C.

La diphényl-4,4 cyclohexadiène-2,5 one-1 peut être préparée selon la méthode de H.E. Zimmermann et D.I.Schuster J. Am. Chem. Soc., 84, 527 (1962).

### EXEMPLE 5

A une solution de 90,3 g de bis(fluoro-3 phényl)-4,4 cyclohexènone et de 123 cm³ de N-butoxyméthyl N-triméthylsilylméthyl benzylamine dans 1000 cm³ de dichlorométhane sec, on ajoute 3 cm³ d'acide trifluoroacétique. Le mélange réactionnel est porté au reflux puis agité 2 heures en laissant revenir la température à 25°C et encore 15 minutes après addition de 60 g de carbonate de potassium. Après filtration et concentration à sec sous pression réduite (2,7 kPa), le résidu cristallisé est battu avec de l'oxyde d'isopropyle,essoré lavé et recristallisé dans 300 cm³ de cyclohexane. Les cristaux sont essorés, lavés 2 fois par 15 cm³ de cyclohexane et séchés pour donner 92 g de benzyl-2 bis(fluoro-3 phényl)-7,7 perhydroisoindolone-4-(3aRS,7aRS) sous la forme de cristaux blancs. P.F. = 124°C.

A une solution de 144,5 g de bis(fluoro-3 phényl)acétaldéhyde dans 500 cm³ d'éther éthylique, on ajoute 50,4 cm³ de butènone, puis après refroidissement à 0°C, goutte à goutte une solution de 13,9 g de potasse dans 89 cm³ d'éthanol. Le mélange réactionnel est agité 2 h à 0°C puis 16 heures à 25 °C, dilué par 300 cm³ d'acétate d'éthyle et 500 cm³ d'eau. La phase aqueuse est lavée par 300 cm³ d'acétate d'éthyle. Les phases organiques réunies sont lavées par 500 cm³ de solution saturée de chlorure de sodium, séchées sur sulfate de magnésium et concentrées sous pression réduite (2,7 kPa). Le résidu est chromatographié (en 2 fois) sur gel de silice (granulomètrie 0,04-0,063 mm, colonnes diamètre 8,5 cm, hauteur 34 cm) en éluant sous une pression de 0,5 bar d'azote par un mélange de cyclohexane et d'acétate d'éthyle (90/10). On obtient 90,3 g de bis(fluoro-3 phényl)-4,4 cyclohexènone, sous forme de cristaux blancs. P.F. = 95°C

Une solution de 156,7 g de bis(fluoro-3 phényl)-1,1 méthoxy-2 éthanol (obtenu par réaction de bromure de (fluoro-3 phényl)magnésium sur du méthoxy-2 acétate de méthyle dans le THF) dans 160 cm³ d'acide formique est chaufée au reflux 16 heures, refroidie et versée dans un mélange de 800 cm³ de solution saturée de carbonate de sodium et de 500 cm³ d'acétate d'éthyle. La phase organique est lavée 2 fois par 500 cm³ d'eau et par 500 cm³ de solution saturée de chlorure de sodium puis séchée et concentrée à sec sous pression réduite (2,7 kPa) pour donner 144,5 g de bis(fluoro-3 phényl)acétaldéhyde sous la forme d'une huile jaune.

### EXEMPLE 6

Une solution de 92,2 g de benzyl-2 bis(fluoro-3 phényl)-7,7 perhydroisoindolone-4-(3aRS,7aRS) dans 860 cm³ de dichloro-1,2 éthane est traitée par 26,3 cm³ de chloroformiate de vinyle et chauffée 3 heures au reflux puis concentrée sous pression réduite (2,7 kPa). Le résidu est chromatographié (en deux fois) sur gel de silice (granulomètrie 0,04-0,063 mm, colonnes de diamètre 8 cm et hauteur 35 cm) en éluant sous une pression de 0,5 bar d'azote par un mélange de cyclohexane et d'acétate d'éthyle (75/25). La meringue obtenue est cristallisée dans de l'oxyde d'isopropyle pour donner 50,3 g de bis(fluoro-3 phényl)-7,7 vinyloxycarbonyl-2 perhydroisoindolone-4-(3aRS,7aRS). P.F. = 152°C.

64,5 g de bis(fluoro-3 phényl)-7,7 vinyloxycarbonyl-2 perhydroisoindoione-4-(3aRS,7aRS) sont traités par 330 cm³ d'une solution 6N d'acide chlorhydrique dans le dioxanne pendant 30 minutes à 25°C. La solution est concentrée à sec sous pression réduite (2,7 kPa) et le résidu repris par 500 cm³ d'éthanol. La solution est chauffée à 60°C pendant 6 heures et agitée 16 heures à 25°C puis concentrée de moitié sous pression réduite (2,7 kPa) et les cristaux formés sont essorés et lavés par de l'oxyde d'isopropyle puis sèchés. On obtient 48,7 g de chlorhydrate de bis(fluoro-3 phényl)-7,7 perhydroisoindolone-4. P.F. = 264°C

### EXEMPLE 7

A une solution de 4,3 g de bis(fluoro-2 phényl)-4,4 cyclohexènone et de 5,8 cm³ de N-butoxyméthyl N-triméthylsilylméthyl benzylamine dans 30 cm³ de dichlorométhane sec, on ajoute 3 gouttes d'acide trifluoroacétique. Le mélange réactionnel est porté au reflux puis agité 16 heures après avoir laissé revenir la température à 25°C. On ajoute 2,5 cm³ de N-butoxyméthyl N-triméthylsilylméthyl benzylamine et 3 gouttes d'acide trifluoroacétique et agite 3 heures au reflux. Le mélange réactionnel est traité par 3 g de carbonate de potassium et agité 15 minutes. Après filtration et concentration à sec sous pression réduite (2,7 kPa), le résidu est chromatographié sur une colonne de gel de silice (granulomètrie 0,04-0,063 mm, diamètre 4 cm, hauteur 32 cm) en éluant sous une pression de 0,5 bar d'azote par un mélange de cyclohexane et d'acétate d'éthyle (85/15) et en recueillant des fractions de 20 cm³. Les fractions 13 à 22 sont réunies et concentrées à sec sous pression réduite (2,7 kPa) pour donner 2,28 g de benzyl-2 bis(fluoro-2 phényl)-7,7 perhydroisoindolone-4-(3aRS,7aRS). P.F. = 138°C.

La bis(fluoro-2 phényl)-4,4 cyclohexènone peut être préparée de la manière suivante : A une solution de 30,8 g de bis(fluoro-2 phényl)acétaldéhyde dans 135 cm³ de diméthoxy-1,2 éthane, on ajoute 26,9 g de carbonate de potassium puis goutte à goutte et après refroidissement à -50°C, 19,9 cm³ de butènone. Le mélange réactionnel est agité 12 h à -50°C puis 6 heures à 25°C, dilué par 250 cm³ d'acétate d'éthyle et 200 cm³ d'eau. La phase organique est lavée 3 fois par 200 cm³ d'eau puis par 200 cm³ de solution saturée de chlorure de sodium, séchées sur sulfate de magnésium et concentrées sous pression réduite (2,7 kPa). Le résidu est chromatographié sur colonne de gel de silice (granulomètrie 0,04-0,063 mm, diamètre 5,5 cm, hauteur 50 cm) en éluant sous une pression de 0,5 bar d'azote par un mélange de cyclohexane et d'acétate d'éthyle (90/10). On obtient 9 g de bis(fluoro-2 phényl)-2,2 oxo-5 hexanal sous forme d'une huilejaune. Une solution de 6,65 g de ce composé dans 100 cm³ de toluène contenant 1,5 g d'acide paratoluènesulfonique est chauffée au reflux pendant 3 heures lavée 2 fois par 100 cm³ d'eau puis par 100 cm³ de solution saturée de chlorure de sodium, séchée sur sulfate de magnésium et concentrée sous pression réduite (2,7 kPa). Le résidu est chromatographié sur une colonne de gel de silice (granulomètrie 0,04-0,063 mm, diamètre 4 cm, hauteur 30 cm) en éluant sous une pression de 0,5 bar d'azote par un mélange de cyclohexane et d'acétate d'éthyle (90/10) et en recueillant des fractions de 15 cm³. Les fractions 21 à 26 sont réunies et concentrées à sec sous pression réduite (2,7 kPa) pour donner 2,83 g de bis(fluoro-2 phényl)-4,4 cyclohexènone, sous forme d'huile jaune.

Spectre RMN du proton (DMSO-dₛ):
2,6 (m, 2H, -CH₂- en 5) ; 2,8 (dd large, 2H, -CH₂- en 6) ; 6,2 (d, 1 H, H en 2) ; 6,9 à 7,4 (m, 9H aromatiques et H en 3).

Le bis(fluoro-2 phényl)acétaldéhyde peut être préparé de la manière suivante :
Une solution de 26,3 g de bis(fluoro-2 phényl)-1,2 oxirane dans 500 cm³ de toluène est traitée goutte à goutte par 7 cm³ d'éthèrate de trifluorure de bore et agitée 2 heures à 25°C puis lavée par 50 cm³ d'eau et 50 cm³ de solution saturée de bicarbonate de sodium. après séchage sur sulfate de magnésium et concentration à sec sous pression réduite (2,7 kPa) on obtient 25 g de bis(fluoro-2 phényl)acétaldéhyde sous la forme d'une huile jaune.

Le bis(fluoro-2 phényl)-1,2 oxirane peut être préparé selon la méthode décrite par V. Mark (J. Am. Chem. Soc., 85, 1884(1963))

### EXEMPLE 8

Une solution de 2,34 g de benzyl-2 bis(fluoro-2 phényl)-7,7 perhydroisoindolone-4-(3aRS,7aRS) dans 100 cm³ de méthanol additionné de 6,2 cm³ d'acide chlorhydrique N est hydrogénée à pression ordinaire en présence de 0,4 g de palladium sur charbon à 10 %, pendant 5 heures à 25 °C. Le milieu réactionnel est filtré et concentré sous pression réduite (2,7 kPa) pour donner 2 g de chlorhydrate de bis(fluoro-2 phényl)-7,7 perhydroisoindolone-4-(3aRS,7aRS) sous la forme d'un solide blanc.
Spectre RMN du proton (DMSO-dₛ): 2 à 2,4 (m, 2H, -CH₂- en 5) ; 2,7 à 3 (m, 4H, -CH₂- en 1 et en 6) ; 3,5 (dd large, 1 H, 1 H en 3) ; 3,7 (dd large, 1 H, H en 3a) ; 3,9 (d large, 1 H, 1 H en 3) ; 4,2 (m, 1 H, H en 7a) ; 7,1 à 8 (m, 8H aromatiques).

### EXEMPLE 9

A une solution de 26,8 g de bis(chloro-3 phényl)-4,4 cyclohexènone et de 33 cm³ de N-butoxyméthyl N-triméthylsilylméthyl benzylamine dans 200 cm³ de dichlorométhane sec, on ajoute 15 gouttes d'acide trifluoroacétique. Le mélange réactionnel est porté au reflux puis agité 16 heures après avoir laissé revenir la température à 25°C et encore 15 minutes après addition de 16 g de carbonate de potassium. Après filtration et concentration à sec sous pression réduite (2,7 kPa), le résidu est chromatographié sur une colonne de gel de silice (granulomètrie 0,04-0,063 mm, diamètre 7 cm, hauteur 40 cm) en éluant sous une pression de 0,5 bar d'azote par un mélange de cyclohexane et d'acétate d'éthyle (75/25) et en recueillant des fractions de 500 cm³. Les fractions 12 à 18 sont réunies et concentrées à sec sous pression réduite (2,7 kPa) pour donner 16,2 g de benzyl-2 bis(chloro-3 phényl)-7,7 perhydroisoindolone-4-(3aRS,7aRS) sous la forme d'une huile jaune. Spectre RMN du proton: (DMSO-dₛ): 1,75 (ddd, 1 H) et 2,1 à 2,45 (m, 3H) : -CH₂- en 5 et en 6) : 2,7 à 2,9 (m, 4H : -CH₂- en 1 et en 3) ; 3,1 (m, 1 H , H en 3a) ; 3,5 (AB, 2H, -CH₂- benzylique) ; 3,8 (dd large, 1 H, H en 7a) ;7,1 à 7,5 (m, 13H aromatiques).

A une solution de 36,9 g de bis(chloro-3 phényl)acétaldéhyde dans 200 cm³ d'éther éthylique, on ajoute 11,3 cm³ de butènone, puis après refroidissement à 0°C, goutte à goutte une solution de 3,1 g de potasse dans 20 cm³ d'éthanol. Le mélange réactionnel est agité 2 h à 0°C puis 16 heures à 25 °C, dilué par 100 cm³ d'acétate d'éthyle et 200 cm³ d'eau. La phase aqueuse est lavée par 100 cm³ d'acétate d'éthyle. Les phases organiques réunies sont lavées 3 fois par 100 cm³ de solution saturée de chlorure de sodium, séchées sur sulfate de magnésium et concentrées sous pression réduite (2,7 kPa). Le résidu est chromatographié sur colonne de gel de silice (granulomètrie 0,04-0,063 mm, diamètre 7 cm, hauteur 42 cm) en éluant sous une pression de 0,5 bar d'azote par un mélange de cyclohexane et d'acétate d'éthyle (90/10). On obtient 27,7 g de bis(chloro-3 phényl)-4,4 cyclohexènone, sous forme d'huile jaune.
Spectre RMN du proton (DMSO-dₛ): 2,3 (dd large, 2H, -CH₂- en 5) ; 2,7 (dd large, 2H, -CH₂- en 6) ; 6,2 (d, 1 H, H en 2) ; 7,2 à 7,4 (m, 8H aromatiques) ; 7,6 (d, 1 H, H en 3).

Une solution de 47 g de bis(chloro-3 phényl)-1,1 méthoxy-2 éthanol (obtenu par réaction de bromure de (chloro-3 phényl)magnésium sur du méthoxy-2 acétate de méthyle dans le THF) dans 44 cm³ d'acide formique est chaufée au reflux 5 heures, refroidie et versée dans un mélange de 500 cm³ de solution saturée de carbonate de sodium et de 300 cm³ d'acétate d'éthyle. La phase organique est lavée 3 fois par 250 cm³ d'eau et par 200 cm³ de solution saturée de chlorure de sodium puis séchée et concentrée à sec sous pression réduite (2,7 kPa) pour donner 36,9 g de bis(chloro-3 phényl)acétaldéhyde sous la forme d'une huile jaune.

### EXEMPLE 10

Une solution de 11,5 g de benzyl-2 bis(chloro-3 phényl)-7,7 perhydroisoindolone-4-(3aRS,7aRS) dans 250 cm³ de dichloro-1,2 éthane est traitée par 2,8 cm³ de chloroformiate de vinyle et chauffée 16 heures au reflux puis concentrée sous pression réduite (2,7 kPa). Le résidu est chromatographié sur une colonne de gel de silice (granulomètrie 0,04-0,063 mm, diamètre 6 cm, hauteur 32 cm) en éluant sous une pression de 0,5 bar d'azote par un mélange de cyclohexane et d'acétate d'éthyle (80/20) et en recueillant des fractions de 25 cm³. Les fractions 19 à 27 sont réunies et concentrées à sec sous pression réduite (2,7 kPa). La meringue obtenue est concrètée dans de l'oxyde d'isopropyle et le précipité est essoré, lavé par de l'oxyde d'isopropyle et séché pour donner 6,7 g de bis(chloro-3 phényl)-7,7 vinyloxycarbonyl-2 perhydroisoindolone-4-(3aRS,7aRS) sous la forme d'un solide blanc.
Spectre RMN du proton (DMSO-dₛ): 2,1 et 2,3 (2 ddd larges, 2H, -CH₂- en 5) ; 2,7 à 3 (m, 4H, -CH₂- en 1 et en 6) ; 3,3 (m, 1 H , H en 3a) ; 3,45 (dd large, 1 H, 1 H en 3) ; 4,1 (m, 2H, H en 7a et 1 H en 3) ; 4,45 et 4,70 (2 d larges, 2H, =CH₂ du vinyle) ; 7,05 ((dd, 1 H, OCH= du vinyle» ;7,2 à 7,7 (m, 8H aromatiques).

1,5 g de bis(chloro-3 phényl)-7,7 vinyloxycarbonyl-2 perhydroisoindolone-4-(3aRS,7aRS) sont traités par 7,4 cm³ d'une solution 6N d'acide chlorhydrique dans le d ioxanne pendant 2 heures à 25°C. La solution est concentrée à sec sous pression réduite (2,7 kPa) et le résidu est chauffé 1 heure en solution dans de l'éthanol à 60°C puis agité pendant 6 heures à 25°C. La solution est concentrée à sec sous pression réduite (2,7 kPa) et la meringue obtenue concrètée dans de l'oxyde d'isopropyle. Le précipité est essoré et lavé par de l'oxyde d'iso- propyle puis séché pour donner 1 g de chlorhydrate de bis(chloro-3 phényl)-7,7 perhydroisoindolone-4.
Spectre RMN du proton (DMSO-dₛ): 2 à 2,4 (m, 2H, -CH₂- en 5) ; 2,55 à 2,9 (m, 2H, -CH₂- en 6) ; 3,3 (dd large, 1 H, 1 H en 3) ; 3,5 (m, 1H, H en 3a) ; 3,85 (d large, 1 H, 1 H en 3) ; 3,95 (m, 1H, H en 7a) ; 7,1à 7,76 (m, 8H aromatiques).

### EXEMPLE 11

A une solution de 16,7 g de bis(tolyl-3)-4,4 cyclohexènone et de 18,7 cm³ de N-butoxyméthyl N-triméthylsilylméthyl benzylamine dans 150 cm³ de dichlorométhane sec, on ajoute 12 gouttes d'acide trifluoroacétique. Le mélange réactionnel est porté au reflux puis agité 3 heures en laissant revenir la température à 25°C et encore 10 minutes après addition de 12 g de carbonate de potassium. Après filtration et concentration à sec sous pression réduite (2,7 kPa), le résidu est chromatographié sur une colonne de gel de silice (granulométrie 0,04-0,063 mm, diamètre 5 cm, hauteur 50 cm) en éluant sous une pression de 0,7 bar d'azote par un mélange de cyclohexane et d'acétate d'éthyle (85/15) et en recueillant des fractions de 25 cm³. Les fractions 14 à 30 sont réunies et concentrées à sec sous pression réduite (2,7 kPa) pour donner 13,9 g de benzyl-2 bis(tolyl-3)-7,7 perhydroisoindolone-4-(3aRS,7aRS) sous la forme d'une huile incolore. Spectre RMN du proton (CDCI₃): 1,98 (ddd, 1 H) et 2,2 à 2,5 (m, 3H) : -CH₂- en 5 et en 6) ; (s , 6H, ArCH₃) ; 2,5 à 3,05 (m, 4H, -CH₂- en 1 et en 3) ; 3,2 (m, 1 H , H en 3a) ; 3,45 et 3,65 (AB, 2H, -CH₂- Ar) ; 3,7 (m, 1 H, H en 7a) ; 6,9 à 7,4 (m, 13H aromatiques).

La bis(tolyl-3)-4,4 cyclohexènone peut être préparée de la manière suivante :
A une solution de 20,4 g de bis(tolyl-3)acétaldéhyde dans 110 cm³ d'éther éthylique, on ajoute 7,23 cm³ de butènone, puis après refroidissement à 0°C, goutte à goutte une solution de 2 g de potasse dans 12,7 cm³ d'éthanol. Le mélange réactionnel est agité 2 heures à 0°C puis 16 heures à 25 °C, dilué par 200 cm³ d'acétate d'éthyle et 200 cm³ d'eau. La phase aqueuse est lavée 2 fois par 250 cm³ d'acétate d'éthyle. Les phases organiques réunies sont lavées 2 fois par 250 cm³ d'eau puis par 250 cm³ de solution saturée de chlorure de sodium, séchées sur sulfate de magnésium et concentrées sous pression réduite (2,7 kPa). Le résidu est chromatographié sur gel de silice (granulomètrie 0,04-0,063 mm, colonne de diamètre 5,4 cm et hauteur 40 cm) en éluant sous une pression de 0,5 bar d'azote par un mélange de cyclohexane et d'acétate d'éthyle (85/15). On obtient 16,7 g de bis(tolyl-3)-4,4 cyclohexènone, sous forme d'huile jaune.
Spectre RMN du proton (CDCI₃): 2,36 (s , 6H, ArCH₃) ; 2,45 (dd large, 2H, -CH₂- en 6) ; 2,72 (dd large, 2H, -CH₂- en 5) ; 6,23 (d, 1 H, H en 2) ; 7 à 7,3 (m, 8H aromatiques) ; 7,34 (d, 1 H, H en 3).

Le bis(tolyl-3)acétaldéhyde peut être préparé de la manière suivante : Une solution de 24,66 g de bis(tolyl-3)-1,1 méthoxy-2 éthanol (obtenu par réaction de bromure de (tolyl-3)magnésium sur du méthoxy-2 acétate de méthyle dans le tétrahydrofuranne) dans 30 cm³ d'acide formique est chauffée au reflux 12 heures, refroidie et versée dans un mélange de 400 cm³ de solution saturée de carbonate de sodium et de 400 cm³ d'acétate d'éthyle. La phase organique est lavée 3 fois par 300 cm³ d'eau et par 300 cm³ de solution saturée de chlorure de sodium puis séchée et concentrée à sec sous pression réduite (2,7 kPa) pour donner 20,45 g de bis(tolyl-3)acétaldéhyde sous la forme d'une huile jaune.

### EXEMPLE 12

Une solution de 13,7 g de benzyl-2 bis(tolyl-3)-7,7 perhydroisoindolone-4-(3aRS,7aRS) dans 150 cm³ de dichloro-1,2 éthane est traitée par 3,7 cm³ de chloroformiate de vinyle et chauffée 3 heures au ref lux puis concentrée sous pression réduite (2,7 kPa). Le résidu est chromatographié sur gel de silice (granulomètrie 0,04-0,063 mm, colonne de diamètre 5,4 cm et hauteur 39 cm) en éluant sous une pression de 0,5 bar d'azote par un mélange de cyclohexane et d'acétate d'éthyle (80/20). Les fractions 23 à 39 sont réunies et concentrées à sec sous pression réduite (2,7 kPa) pour donner 7,4 g de bis(tolyl-3)-7,7 vinyloxycarbonyl-2 perhydroisoindolone-4-(3aRS,7aRS). sous la forme d'une meringue blanche.

Spectre RMN du proton (DMSO-dₛ/AcOD 90/10):
A température ordinaire on observe le mélange des 2 rotamères. 1,95 à 2,4 (m, 2H, -CH₂- en 5) ; 2,27 et 2,32 (2s , 6H, ArCH₃) ; 2,4 à 2,95 (m, 4H, -CH₂- en 1 et en 6) ; 3,2 à 3,5 (m, 2H , H en 3a et 1 H en 3) ; 4,03 (m, 1 H H en 7a) ; 4,09 et 4,16 (2d larges, 1 H, H en 3) ; 4,35 à 4.85 (4d larges, 2H, =CH₂ du vinyle) ; 6,9 à 7,5 (m, 9H, aromatiques et OCH= du vinyle).

7,4 g de bis(tolyl-3)-7,7 vinyloxycarbonyl-2 perhydroisoindolone-4-(3aRS,7aRS) sont traités par 39 cm³ d'une solution 6N d'acide chlorhydrique dans le dioxanne pendant 30 minutes à 25°C. La solution est concentrée à sec sous pression réduite (2,7 kPa) et le résidu repris par 100 cm³ d'éthanol. La solution est chauffée à 60°C pendant 2 heures et agitée 16 heures à 25°C puis concentrée à sec sous pression réduite (2,7 kPa). Le résidu est concrèté par de l'oxyde d'isopropyle, le solide est lavé essoré et séché. On obtient 6,36 g de chlorhydrate de bis(tolyl-3)-7,7 perhydroisoindolone-4. sous la forme d'un solide jaune.

Spectre de RMN du proton (DMSO-d₆/AcOD 90/10):
1,95 à 2,35 (m, 2H, -CH₂- en 5) ; 2,24 et 2,3 (2s , 6H, ArCH₃) ; 2,4 à 2,9 (m, 4H, -CH₂- en 6 et en 1) ; 3,3 (dd large, 1 H, 1 H en 3) ; 3,48 (m, 1 H, H en 3a) ; 3,85 (d large, 1 H, 1 H en 3) ; 3,90 (m, 1 H, H en 7a) ; 6,9 à 7,4 (m, 8H aromatiques).

### EXEMPLE 13

Une solution de 25 g de diphényl-4,4 cyclohéxene-2 one-1 et de 2,5 g de benzyl-3 oxazolidinone-5 dans 100 cm³ de toluène sec est chauffée au reflux pendant 2 heures 30 minutes. Le mélange réactionnel est concentré à sec sous pression réduite (2,7 kPa) Le résidu est chromatographié sur une colonne de gel de silice (0,2-0,063 mm, diamètre 4,5 cm, hauteur23 cm) en éluantsous une pression de 0,5 bard'azote par un mélange de cyclohexane et d'acétate d'éthyle (80/20 en volumes) et en recueillant des fractions de 50 cm³. Les fractions 13 à 17 sont réunies et concentrées à sec sous pression réduite (2,7 kPa) pour donner 0,9 g de benzyl-2 diphényl-7,7 perhydroisoindolone-4-(3aRS,7aRS) sous la forme de cristaux blancs, fondant à 132°C.

La benzyl-3 oxazolidinone-5 peut être préparée selon la méthode de M. Joucla et J. Mortier, Bull. Soc. Chim. Fr., 579 (1988).

### EXEMPLE 14

A une solution portée au reflux de 0,14 g de paraformaldéhyde dans 20 cm³ de solution aqueuse à 2% d'acide sulfurique on ajoute une solution de 0,41 g de benzylaminométhyl-6 diphényl-7,7 dioxa-1,4 spiro [4,5] décane-(R,S) dans 0,4 cm³ d'éthanol et on poursuit le chauffage à reflux pendant48 heures. Le mélange réactionnel est refroidi à +25_{°}C, alcalinisé avec 5 cm³ de solution aqueuse de soude 4N, extrait avec 4 fois 40 cm³d'acétate d'éthyle, les phases organiques sont réunies, lavées avec 100 cm³ d'eau distillée, séchées sur sulfate de magnésium, filtrées et concentrées à sec sous pression réduite (2,7 kPa). Le résidu est chromatographié sur colonne de gel de silice avec légère supression d'azote (0,04-0,063 mm, diamètre 2 cm, hauteur 15 cm) en éluant par un mélange de cyclohexane et d'acétate d'éthyle (60/40 en volumes) et en recueuillant des fractions de 20 cm³. Les fractions 4 et 5 sont réunies et concentrées à sec sous pression réduite (2,7 kPa). On obtient 0,23 g de benzyl-2 diphényl-7,7 perhydroisoindolone-4-(3aRS,7aRS) sous forme de cristaux blancs, fondant à 132°C.

Le benzylaminométhyl-6 diphényl-7,7 dioxa-1,4 spiro [4,5] décane-(RS) peut être préparé de la manière suivante :
A une suspension de 1,9 g d'hydrure de lithium et d'aluminium dans 60 cm³de tétrahydrofuranne anhydre, refroidie à +5_{°}C, on ajoute goutte à goutte, pendant 1 heure 30 minutes, et en maintenant la température du mélange réactionnel à +5_{°}C, une solution de 8,1 g de benzamidométhyl-6 diphényl-7,7 1,4-dioxaspiro [4,5] décane-(RS) dans 150 cm³ de tétrahydrofuranne anhydre, puis on porte au reflux pendant 24 heures. Le mélange réactionnel est ensuite refroidi à +5°C, traité par 2,1 cm³ d'eau distillée, puis par 1,9 cm³ de solution aqueuse de soude 5N, puis par 5,8 cm³ d'eau distillée, filtré et concentré à sec sous pression réduite (2,7 kPa). Le résidu est chromatographié sur colonne de gel de silice avec une légère supression d'azote (0,04-0,063 mm, diamètre 5 cm, hauteur 29 cm) en éluant par de l'acétate d'éthyle et en recueillant des fractions de 120 cm³. les fractions 3 à 12 sont réunies et concentrées à sec sous pression réduite (2,7 kPa). On obtient 6,6 g de benzylaminométhyl-6 diphényl-7,7 dioxa-1,4 spiro [4,5] décane-(RS) sous forme d'huile jaune.

Le benzamidométhyl-6 diphényl-7,7 dioxa-1,4 spiro [4,5] décane-(RS) peut être préparé de la manière suivante :
A une solution de 16,2 g d'aminométhyl-6 diphényl-7,7 dioxa-1,4 spiro [4,5] décane-(RS) et de 5,6 g de triéthylamine dans 150 cm³ de dichlorométhane anhydre, refroidie à +5_{°}C, on ajoute goutte à goutte, pendant 40 minutes, et en maintenant la température du mélange réactionnel à +5_{°}C, une solution de 7,7 g de chlorure de benzoyle dans 10 cm³ de dichlorométhane anhydre. Le mélange réactionnel est ensuite agité pendant 4 heures à +25_{°}C, puis lavé avec 125 cm³ d'eau distillée refroidie à +5_{°}C, séché sur sulfate de magnésium, filtré et concentré à sec sous pression réduite (2,7 kPa). Le résidu est recristallisé dans 50 cm³ d'un mélange d'acétonitrile et d'oxyde d'isopropyle (50/50 en volumes). Les cristaux sont essorés et séchés. On obtient 16 g de benzamidométhyl-6 diphényl-7,7 dioxa-1,4 spiro [4,5] décane-(RS) sous forme de cristaux blancs fondant à 162°C.

L'aminométhyl-6 diphényl-7,7 dioxa-1,4 spiro [4,5] décane-(RS) peut être préparé de la manière suivante : A une suspension de 8,13 g d'hydrure de lithium et d'aluminium dans 250 cm³ de tétrahydrofuranne anhydre, refroidie à +5_{°}C, on ajoute goutte à goutte pendant 1 heure une solution de 63 g de nitrométhyl-6 diphényl-7,7 dioxa-1,4 spiro[4,5] décane-(RS) dans 300 cm³ de tétrahydrofuranne anhydre en maintenant la température du mélange réactionnel à +5_{°}C, puis on porte au reflux pendant 2 heures. Le mélange réactionnel est ensuite refroidi à +5_{°}C, traité par 8,93 cm³ d'eau distillée, puis par 8 cm³ de solution aqueuse de soude 5 N, puis par 25 cm³ d'eau distillée, filtré et concentré à sec sous pression réduite (2,7 kPa). Le résidu est chromatographié sur colonne de gel de silice avec légère surpression d'azote (0,04-0,063 mm, diamètre 6,5 cm, hauteur 45 cm) en éluant par un mélange de dichlorométhane et de méthanol (90/10 en volumes) et en recueillant des fractions de 120 cm³. Les fractions 10 à 31 sont réunies et concentrées à sec sous pression réduite (2,7 kPa). On obtient 16,5 g d'aminométhyl-6 diphényl-7,7 dioxa-1,4 spiro[4,5] décane-(RS) sous forme d'huile orange.

Le nitrométhyl-6 diphényl-7,7 dioxa-1,4 spiro [4,5] décane-(RS) peut être préparé de la manière suivante : Une solution de 69 g de nitrométhyl-3 diphényl-4,4 cyclohexanone et 30,48 g d'éthylène glycol et 106,8 g de chlorotriméthylsilane dans 1 litre de chlorure de méthylène anhydre est portée au reflux pendant 2 heures, puis refroidie à +25_{°}C, lavée par 600 cm³ d'une solution aqueuse saturée d'hydrogénocarbonate de sodium, puis par 300 cm³ d'une solution aqueuse saturée de chlorure de sodium, séchée sur sulfate de magnésium, filtrée et concentrée à sec sous pression réduite (2,7 kPA). Le résidu est chromatographié sur colonne de gel de silice avec légère surpression d'azote (0,04-0,063 mm, diamètre 6,5 cm, hauteur 45 cm) en éluant par un mélange de cyclohexane et d'acétate d'éthyle (90/10 en volumes) en recueillant des fractions de 500 cm³. Les fractions 1 à 3 sont réunies et concentrées à sec sous pression réduite (2,7 kPa). On obtient 63,3 g de nitrométhyl-6 diphényl-7,7 dioxa-1,4 spiro [4,5] décane-(RS) sous forme de cristaux blancs, fondant à 148°C.

La nitrométhyl-3 diphényl-4,4 cyclohexanone-(RS) peut être préparée de la manière suivante :
A une solution de 60 g de diphényl-4,4 cyclohexène-2 one et 14,75 g de nitrométhane dans 400 cm³ de méthyl-2 propanol-2 et 200 cm³ de tétrahydrofuranne anhydre on ajoute 11,45 g d'une solution d'hydroxyde de ben- zyltriméthylammonium dans le méthanol et on agite le mélange réactionnel à +25_{°}C pendant 144 heures : un solide cristallin précipite lentement. On filtre cette suspension, lave les cristaux par 50 cm³d'éther de pétrole refroidi à 0°C, essore et sèche. On obtient 69,1 g de nitrométhyl-3 diphényl-4,4 cyclohexanone-(RS) sous forme de cristaux blancs fondant à 164°C.

### EXEMPLE 15

A une solution portée au reflux de 0,09 g de paraformaldéhyde dans 5 cm³ de solution aqueuse à 2 % d'acide sulfurique on ajoute une solution de 1 g d'aminométhyl-6 diphényl-7,7 dioxa-1,4 spiro [4,5] décane-(RS) dans 2 cm³ d'éthanol et on poursuit le chauffage à reflux pendant 48 heures. Le mélange réactionnel est ensuite refroidi à +5_{°}C, alcalinisé avec une solution aqueuse de soude 4N, extrait avec 3 fois 50 cm³ de dichlorométhane, les phases organiques sont réunies, lavées avec 100 cm³ d'eau distillée, séchées sur sulfate de magnésium, filtrées et concentrées à sec sous pression réduite (2,7 kPa). La résidu est dissous dans 8 cm³ d'acétone, cette solution est acidifiée avec 2 cm³ d'éther chlorhydrique 3,6 N, et concentrée à sec sous pression réduite (2,7 kPa). Le résidu est chromatographié sur colonne de gel de silice avec légère surpression d'azote (0,04-0,063 mm, diamètre 2,5 cm, hauteur 35 cm) en éluant par un mélange de dichlorométhane et de méthanol (85/15 en volumes) et en recueillant des fractions de 15 cm³. La fraction 9 est concentrée à sec sous pression réduite (2,7 kPa), dissoute dans 10 cm³ d'eau, cette solution aqueuse est alcalinisée à +5_{°}C par une solution aqueuse de soude 4 N, extraite avec 3 fois 30 cm³ de dichlorométhane, les phases organiques sont réunies, lavées avec 50 cm³ d'eau distillée, séchées sur sulfate de magnésium, filtrées et concentrées à sec sous pression réduite. On obtient 0,07 g de diphényl-7,7 perhydroisoindolone-4-(3aRS,7aRS) sous forme d'une meringue blanche ;

Spectre RMN du proton :
2,15 et 2,4 (2 Mt, respectivement 1 H chacun, -CH₂- en 6) ; 2,75 (Mt, 4H, -CH₂- en 1 et -CH₂- en 5) ; 3,3 à 3,6 (Mt, 2H, 1 H du -CH₂- en 3 et '^{CH-} en 3a) ; 3,95 à 4,2 (Mt, 2H, 1 H du -CH₂- en 3 et 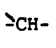 en 7a) ; 7 à 7,5 (Mt, 10H, aromatiques).
   Spectre infra-rouge (CHBr₃) bandes caractéristiques (cm-¹) ;
   3350, 3100-3000, 3000-2800, 1705, 1600, 1580, 1495, 1460, 1445, 755.

### EXEMPLE 16

Une solution de 1,7 g de diphényl-4,4 formyl-2 nitrométhyl-3 cyclohexanone dans 50 cm³ d'acide acétique est hydrogénée en présence de 0,2 g de palladium sur charbon à 10% à 50°C et sous une pression de 50 bars. Après 5 heures de réaction le mélange réactionnel est filtré, puis concentré à sec sous pression réduite (2,7 kPa) ; le résidu est repris dans 100 cm³ d'acétate d'éthyle et cette solution est lavée par 100 cm³ d'eau et 100 cm³ de solution saturée de chlorure de sodium puis sèchée sur sulfate de magnésium et traitée par une solution de gaz chlorhydrique dans l'oxyde d'isopropyle. L'huile formée est décantée et cristallisée dans 30 cm³ d'acétone. Les cristaux sont essorés, lavés par de l'acétone et séchés. On obtient 0,55 g de chlorhydrate de diphényl-7,7 perhydroisoindolone-4-(3aRS,7aRS), fondant à 270°C avec décomposition.

La diphényl-4,4 formyl-2 nitrométhyl-3 cyclohexanone peut être préparée de la manière suivante:
Une solution de 29 g de diphényl-4,4 formyl-2 cyclohexène-2 one-1, 5,7 cm³ de nitrométhane dans un mélange de 250 cm³ de tétrahydrofuranne et de 500 cm³ de tert-butanol est traitée par 6,93 cm³ d'une solution à 35 % d'hydroxyde de benzyl triméthyl ammonium dans le méthanol et agitée à 20°C pendant 18 heures. Le mélange réactionnel est dilué par 500 cm³ d'acétate d'éthyle et 2000 cm³ d'eau et acidifié à pH 2 par de l'acide chlorhydrique 4N. La phase aqueuse est extraite par 200 cm³ d'acétate d'éthyle et les phases organiques réunies sont lavées par 500 cm³ d'eau et 250 cm³ de solution saturée de chlorure de sodium puis séchées sur sulfate de magnésium et concentrées à sec sous pression réduite (2,7 kPa). Le résidu cristallisé est agité dans 100 cm³ de mélange de cyclohexane et d'acétate d'éthyle (80/20 en volumes), les cristaux sont essorés, lavés par 20 cm³ du même mélange et 2 fois par 50 cm³ d'oxyde d'isopropyle. On obtient 24,7 g de diphényl-4,4 formyl-2 nitrométhyl-3 cyclohexanone sous la forme de cristaux crème, fondant à 188°C.

La diphényl-4,4 formyl-2 cyclohexène-2-one-1 peut être préparée de la manière suivante :
Une solution de 44,5 g de diphényl-4,4 formyl-2 cyclohexanone et de 38 g de dichloro-2,3 dicyano-5,6 ben- zoquinone-1,4 dans 650 cm³ de dioxanne est agitée 30 minutes à 25°C puis refroidie à 0°C ; le solide est éliminé par filtration et le filtrat est concentré à sec sous pression réduite (2,7 kPa). Le résidu est chromatographié sur une colonne de gel de silice (0,2-0,06 mm, diamètre 6 cm, hauteur 63 cm) en éluant par un mélange de cyclohexane et d'acétate d'éthyle (80/20 en volumes) et en recueillant des fractions de 50 cm³. Les fractions 7 à 12 sont réunies et concentrées à sec sous pression réduite (2,7 kPa). Le résidu cristallin est battu avec 200 cm³ d'oxyde d'isopropyle, les cristaux obtenus sont essorés et lavés 2 fois par 50 cm³ d'oxyde d'isopropyle et séchés. On obtient 12 g de diphényl-4,4 formyl-2 cyclohexène-2 one-1, fondant à 126°C.

La diphényl-4,4 formyl-2 cyclohexanone peut être préparée de la manière suivante :
On ajoute 40 g de diphényl-4,4 cyclohexanone à une solution de 66,5 g de tert-butylate de potassium dans 500 cm³ de tert-butanol puis on additionne goutte à goutte une solution de 47,6 cm³ de formiate d'éthyle dans 500 cm³ de tert-butanol. Le mélange réactionnel est agité à 50 °C pendant 8 heures puis refroidi et dilué par 3000 cm³ d'eau et 750 cm³ d'acétate d'éthyle et acidifié à pH 2 par de l'acide chlorhydrique 4N. La phase aqueuse est extraite 2 fois par 250 cm³ d'acétate d'éthyle et les phases organiques réunies sont lavées par 250 cm³ d'eau et 250 cm³ de solution saturée de chlorure de sodium puis séchées sur sulfate de magnésium et concentrées à sec sous pression réduite (2,7 kPa). On obtient 48 g de diphényl-4,4 formyl-2 cyclohexanone utilisée sans purification pour la poursuite de la synthèse.

### EXEMPLE 17

A une suspension de 200 g de chlorhydrate de diphényl-7,7 perhydroisoindolone-4-(3aRS,7aRS) dans 2000 cm³ d'acétate d'éthyle , on ajoute lentement, sous agitation, 500 cm³ de soude aqueuse 4H ; l'agitation est poursuivie jusqu'à disparition du produit de départ. La solution organique est lavée par 250 cm³ d'eau distillée, par 250 cm³ d'une solution aqueuse saturée en chlorure de sodium, séchée sur sulfate de magnésium et filtrée. Ala solution ainsi obtenue, on ajoute, sous agitation, une solution de 92,8 g d'acide L-(+) mandélique dans 1000 cm³ d'acétate d'éthyle ; après 4 heures d'agitation, les cristaux obtenus sont essorés, lavés par 250 cm³ d'acétate d'éthyle (2 fois) et séchés. Les cristaux sont repris par 2000 cm³ d'eau distillée ; le mélange est chauffé au reflux, sous agitation, pendant 15 minutes ; les cristaux insolubles sont essorés, lavés par 100 cm³ d'eau distillée (2 fois) et séchés. Ils sont recristallisés dans un mélange de 1100 cm³ d'acétonitrile et de 500 cm³ d'eau distillée; les cristaux obtenus sont essorés, lavés par 40 cm³ d'acétonitrile (3 fois) et séchés. On obtient 80 g de (L)- mandélate de diphényl-7,7 perhydroisoindolone-4 (3aR,7aR) ;
[α]_{D}²⁰ = -₁₆₄0 (c=1, méthanol).

A 80 g de (L)-mandélate de diphényl-7,7 perhydroisoindolone-4-(3aR,7aR), on ajoute 400 cm³ de soude aqueuse 1 N et 600 cm³ d'acétate d'éthyle ; le mélange est agité à température ambiante jusqu'à disparition du produit de départ ; la solution organique est lavée par 250 cm³ d'eau distillée, par 250 cm³ d'une solution aqueuse saturée en chlorure de sodium , séchée sur sulfate de magnésium et filtrée ; elle est acidifiée, sous agitation, par addition de 30 cm³ d'acide chlorhydrique 9N ; les cristaux obtenus sont essorés , lavés par 50 cm³ d'acétate d'éthyle (2 fois) , par 50 cm³ d'oxyde d'isopropyle et séchés . On obtient 52,3 g de chlorhydrate de diphényl-7,7 perhydroisoindolone-4(3aR,7aR), fondant à 270°C avec décomposition ;
[α]_{D}²⁰ = -282_{°} (c=0,5, méthanol).

### EXEMPLE DE REFERENCE 1

A une solution de 1,34 g d'acide phénylacétique dans 30 cm³ de dichlorométhane sec, refroidie à +5_{°}C, on ajoute 1,7 g de N,N'-carbonyldiimidazole. On agite 1 heure à +5_{°}C puis on ajoute une solution de 3,27 g de chlorhydrate de diphényl-7,7 perhydroisoindolone-4 et de 1,7 cm³ de triéthylamine dans 30 cm³ de dichlorométhane. Le mélange réactionnel est agité 1 heure à +5_{°}C, puis 1 heure à 20°C. Le mélange réactionnel est lavé par 50 cm³ d'une solution aqueuse saturée en hydrogénocarbonate de sodium, séché sur sulfate de magnésium, filtré et concentré à sec sous pression réduite (2,7 kPa). Le résidu est cristallisé dans 15 cm³ d'acétonitrile. Les cristaux sont essorés, lavés par 10 cm³ d'oxyde d'isopropyle et séchés. On obtient 2,7 g de diphényl-7,7 (phénylacétyl)-2 perhydroisoindolone-4-(3aRS,7aRS) fondant à 216°C.

### EXEMPLE DE REFERENCE 2

A une solution de 10 g de chlorhydrate de diphényl-7,7 perhydroisoindolone-4-(3aR,7aR) dans 80 cm³ de dichlorométhane sec, refroidie à +5_{°}C, on ajoute goutte à goutte une solution de 4 cm³ de chlorure de phénylacétyle et de 8,6 cm³ de triéthylamine dans 10 cm³ de dichlorométhane. Le mélange réactionnel est agité 2 heures à +5_{°}C, puis 20 heures à 20°C. Le mélange réactionnel est traité par 30 cm³ d'une solution aqueuse saturée en hydrogénocarbonate de sodium ; la phase organique est lavée par 50 cm³ d'eau distillée (2 fois), séchée sur sulfate de magnésium, filtrée et concentrée à sec sous pression réduite (2,7 kPa). Le résidu est cristallisé dans 15 cm³ d'acétonitrile. Les cristaux sont essorés, lavés par 10 cm³ d'acétonitrile, par 10 cm³ d'oxyde d'isopropyle et séchés. On obtient 5,7 g de diphényl-7,7 (phénylacétyl)-2 perhydroisoindolone-4-(3aR,7aR), fondant à 173°C ;
[a]D20= -282° (c=1, méthanol).

### EXEMPLE DE REFERENCE 3

Aune solution de 20 g de diphényl-7,7 phénylacétyl-2 perhydroisoindolinone-4-(3aRS,7aRS) dans 50 cm³ de dichlorométhane, on ajoute 10,45 g de tétrafluoroborate de triéthyloxonium. Le mélange réactionnel est laissé sous agitation 20 heures à température ambiante. Le précipité obtenu est essoré, lavé par 10 cm³ de dichlorométhane anhydre, par 10 cm³ d'éther anhydre et séché ; on obtient 11,1 g de tétrafluoroborate d'[(éthoxy-1 phényl-2) éthylidène]-2 oxo-4 diphényl-7,7 perhydroisoindolium-(3aRS,7aRS) sous la forme d'une poudre blanche utilisée à l'état brut pour les manipulations suivantes.

A une suspension agitée et refroidie à -20°C de 3,75 g de tetrafluoroborate d'[(éthoxy-1 phényl-2) éthyli- dène]-2 oxo-4 diphényl-7,7 perhydroisoindolium-(3aRS,7aRS) dans 30 cm³ de dichlorométhane anhydre, on ajoute 8,8 cm³ d'une solution de dichlorométhane 0,8 N en ammoniac. On laisse le mélange réactionnel revenir à température ambiante et on poursuit l'agitation 5 heures. Le mélange réactionnel est traité par 30 cm³ d'une solution aqueuse à 10% de carbonate de potassium. Le précipité présent est éliminé parfiltration, puis la phase organique est lavée par 15 cm³ d'eau distillée, par 15 cm³ d'une solution aqueuse saturée en chlorure de sodium, séchée sur sulfate de magnésium et concentrée à sec sous pression réduite (2,7 kPa). Le résidu est recristallisé dans 22 cm³ d'acétonitrile. Les cristaux obtenus sont essorés et séchés. On obtient 1,3 g d'(a-iminophénéthyl)-2 diphényl-7,7 perhydroisoindolone-4-(3aRS,7aRS ) fondant à 202°C.

### EXEMPLE DE REFERENCE 4

En opérant de manière analogue à l'exemple de référence 3, à partir du produit obtenu à l'exemple de référence 2, on prépare les produits suivants :
- (a-benzyliminophénéthyl)-2 diphényl-7,7 perhydroisoindolone-4-(3aRS,7aRS) fondant à 165°C.
- [a-(fluoro-2 benzyl) iminophénéthyl]-2 diphényl-7,7 perhydroisoindolone-4-(3aRS,7aRS) fondant à 160°C.
- diphényl-7,7 [a-(thiényl-2 méthyl) iminophénéthyl]-2 perhydroisoindolone-4-(3aRS,7aRS) fondant à 114°C.
- diphényl-7,7 [a-(pyridyl-2 méthyl) iminophénéthyl]-2 perhydroisoindolone-4-(3aRS,7aRS) fondant à 180°C.

### EXEMPLE DE REFERENCE 5

A une solution de 1,06 g d'acide hydroxy-2 phénylacétique dans 30 cm³ de dichlorométhane sec, refroidie à +5_{°}C, on ajoute 1,14 g de N,N'-carbonyldiimidazole. On agite 30 minutes à +5_{°}C puis on ajoute une solution de 2,23 g de chlorhydrate de diphényl-7,7 perhydroisoindolone-4-(3aRS,7aRS) et de 1,96 cm³ de triéthylamine dans 20 cm³ de dichlorométhane. Le mélange réactionnel est agité à 20°C pendant 16 heures, puis lavé par 100 cm³ d'eau (2 fois), séché sur sulfate de magnésium, filtré et concentré à sec sous pression réduite (2,7 kPa).

Le résidu est chromatographié sur une colonne de gel de silice (0,2-0,063 mm, diamètre 4 cm, hauteur 40 cm) en éluant sous une pression de 0,7 bar d'azote par un mélange de cyclohexane et d'acétate d'éthyle (70/30 en volumes) et en recueillant des fractions de 125 cm³. Les fractions 4 à 11 sont réunies et concentrées à sec sous pression réduite (2,7 kPa). Le résidu est cristallisé dans un mélange de 15 cm³ d'acétonitrile et 30 cm³ d'oxyde d'isopropyle. Les cristaux sont essorés, lavés à l'oxyde d'isopropyle et séchés. On obtient 0,8 g de diphényl-7,7 [(hydroxy-2 phényl) acétyl]-2 perhydroisoindolone-4-(3aRS,7aRS) sous la forme de cristaux blancs, fondant à 232°C.

### EXEMPLE DE REFERENCE 6

Aune solution de 1,16 g d'acide méthoxy-2 phénylacétique dans 30 cm³ de dichlorométhane sec, refroidie à +5_{°}C, on ajoute 1,13 g de N,N'-carbonyldiimidazole. On agite 15 minutes à +5_{°}C puis on ajoute une solution de 2,28 g de chlorhydrate de diphényl-7,7 perhydroisoindolone-4-(3aRS,7aRS) et de 1,96 cm³ de triéthylamine dans 20 cm³ de dichlorométhane. Le mélange réactionnel est agité à 20°C pendant 16 heures, puis lavé par 150 cm³ d'eau (2 fois), séché sur sulfate de magnésium, filtré et concentré à sec sous pression réduite (2,7 kPa). La meringue obtenue est cristallisée dans un mélange de 30 cm³ d'acétonitrile et 30 cm³ d'oxyde d'iso- propyle. Les cristaux sont essorés, lavés par 25 cm³ oxyde d'isopropyle et séchés. On obtient 2 g de diphényl-7,7 [(méthoxy-2 phényl) acétyl]-2 perhydroisoindolone-4-(3aRS,7aRS) sous la forme de cristaux blancs, fondant à 163°C.

### EXEMPLE DE REFERENCE 7

A une solution de 5 g de diphényl-7,7 [(méthoxy-2 phényl) acétyl]-2 perhydroisoindolone-4-(3aRS,7aRS) dans 10 cm³ de dichlorométhane, on ajoute 2,35 g de tétrafluoroborate de triéthyloxonium. Le mélange réactionnel est laissé sous agitation 20 heures à température ambiante ; il est alors traité par 100 cm³ d'éther, et le précipité obtenu est essoré, lavé par 100 cm³ d'éther, et séché. On obtient 5,75 g tétrafluoroborate d'[(éthoxy-1 (méthoxy-2 phényl)-2) éthylidène]-2 oxo-4 diphényl-7,7 perhydroisoindolium-(3aRS,7aRS) sous forme d'une poudre jaune utilisée à l'état brut pour la manipulation suivante.

Aune suspension agitée et refroidie à -10°C de 5,7 g de tétrafluoroborate d'[(éthoxy-1 (méthoxy-2 phényl)-2) éthylidène]-2 oxo-4 diphényl-7,7 perhydroisoindolium(3aRS,7aRS) dans 15 cm³ de dichlorométhane anhydre, on ajoute 1,3 cm³ d'une solution éthanolique d'ammoniac 5,4N. On laisse ensuite le mélange réactionnel revenir à température ambiante et on poursuit l'agitation 20 heures. Le mélange réactionnel est dilué par 20 cm³ de dichlorométhane et traité par 20 cm³ d'une solution aqueuse à 10% de carbonate de potassium. Le précipité présent est éliminé par filtration, puis la phase organique est lavée par 25 cm³ d'eau distillée, par 25 cm³ d'une solution aqueuse saturée en chlorure de sodium, séchée sur sulfate de magnésium et concentrée à sec sous pression réduite (2,7 kPa). Le résidu est cristallisé dans 10 cm³ d'acétonitrile. Les cristaux obtenus sont essorés, lavés par 10 cm³ d'acétonitri le et séchés. On obtient 1 g d' [imino-1 (méthoxy-2 phényl)-2 éthyl]-2 diphényl-7,7 perhydroisoindolone-4-(3aRS,7aRS) fondant à une température supérieure à 260°C.

### EXEMPLE DE REFERENCE 8

A une solution de 1 g d'acide méthoxy-2 phénylacétique dans 30 cm³ de dichlorométhane sec, refroidie à +5_{°}C, on ajoute 1 g de N,N'-carbonyldiimidazole. On agite 40 minutes à +5_{°}C puis on ajoute une solution de 2 g de chlorhydrate de diphényl-7,7 perhydroisoindolone-4-(3aR,7aR) et de 1,7 cm³ de triéthylamine dans 40 cm³ de dichlorométhane. Le mélange réactionnel est agité à 20°C pendant 16 heures, puis lavé par 50 cm³ d'eau (2 fois), séché sur sulfate de magnésium filtré et concentré à sec sous pression réduite (2,7 kPa). Le résidu est chromatographié sur une colonne de gel de silice (0,2-0,063 mm, diamètre 1,8 cm, hauteur 13 cm) en éluant par de l'acétate d'éthyle et en recueillant des fractions de 25 cm³. Les fractions 3 à 5 sont réunies et concentrées à sec sous pression réduite (2,7 kPa). Le résidu est cristallisé dans un mélange de 5 cm³ d'acétonitrile et 10 cm³ d'oxyde d'isopropyle. Les cristaux sont essorés, lavés par 25 cm³ oxyde d'isopropyle et séchés. On obtient 1,7 g de (-)-diphényl-7,7 [(méthoxy-2 phényl) acétyl]-2 perhydroisoindolone-4-(3aR,7aR) sous la forme de cristaux blancs, fondant à 200°C ;
[α]_{D}²⁰= - 274° (c=0,49 , acide acétique).

### EXEMPLE DE REFERENCE 9

A une solution de 7,7 g de diphényl-7,7 [(méthoxy-2 phényl) acétyl]-2 perhydroisoindolone-4-(3aR,7aR) dans 13 cm³ de dichlorométhane anhydre, on ajoute 4 g de tétrafluoroborate de triéthyloxonium.Le mélange réactionnel est laissé sous agitation 20 heures à tempé+rature ambiante ; on refoidit ensuite le mélange à - 15°C, puis on ajoute 2,6 cm³ d'une solution éthanolique d'ammoniac 5,4N. On laisse le mélange réactionnel revenir à température ambiante et on poursuit l'agitation 5 heures et demi. Le mélange réactionnel est traité par 20 cm³ d'une solution aqueuse de carbonate de potassium à 10% ; le précipité formé est essoré et lavé par 10 cm³ de dichlorométhane. Les phases organiques sont réunies, séchées sur sulfate de magnésium et concentrées à sec sous pression réduite (2,7 kPa). Le résidu est cristallisé dans 10 cm³ d'acétonitri le ; les cristaux obtenus sont essorés, lavés par 5 cm³ d'acétonitrile, par 10 cm³ d'oxyde d'isopropyle et séchés. Ils sont ensuite chromatographiés sur une colonne de gel d'alumine neutre Péchiney CBT1 (diamètre 4,5 cm, hauteur 28 cm), en éluant par 200 cm³ d'un mélange de dichloro-1,2 éthane et de méthanol (98/2 en volumes), puis par un mélange de dichloro-1,2 éthane et de méthanol (90/10 en volumes) et en recueillant des fractions de 25 cm³.Les fractions 8 à 31 sont réunies et concentrées à sec sous pression réduite (2,7 kPa). Le résidu est cristallisé dans 10 cm³ d'acétonitrile ; les cristaux obtenus sont essorés et séchés. On obtient 1,6 g d'[imino-1 (méthoxy-2phényl)-2 éthyl]-2 diphényl-7,7 perhydroisoindolone-4-(3aR,7aR), fondant à 190°C ;
[_{a]}p²° ^{_} = -₂₅₄0 (c=1 , méthanol).

### EXEMPLE DE REFERENCE 10

A une solution de 2,51 g d'acide (tert-butoxycarbonylamino-2 phényl) acétique dans 30 cm³ de dichlorométhane sec, refroidie à +5_{°}C, on ajoute 1,62 g de N,N'-carbonyldiimidazole. On agite 45 minutes à +5_{°}C puis on ajoute une solution de 3,27 g de chlorhydrate de diphényl-7,7 perhydroisoindolone-4-(3aRS,7aRS) et de 2,8 cm³ de triéthylamine dans 30 cm³ de dichlorométhane. Le mélange réactionnel est agité à 20°C pendant 16 heures, puis lavé par 100 cm³ d'eau (3 fois), séché sur sulfate de magnésium, filtré et concentré à sec sous pression réduite (2,7 kPa). Le résidu est chromatographié sur une colonne de gel de silice (0,2-0,063 mm, diamètre 4 cm, hauteur 40 cm) en éluant sous une pression de 0,7 bar d'azote par un mélange de cyclohexane et d'acétate d'éthyle (60/40 en volumes) et en recueillant des fractions de 125 cm³. Les fractions 22 à 34 sont réunies et concentrées à sec sous pression réduite (2,7 kPa) pour donner 1,5 g de [(tert-butoxycarbonylamino-2 phényl) acétyl]-2 diphényl-7,7 perhydroisoindolone-4-(3aRS,7aRS) sous la forme d'une meringue jaune.

On traite 1,5 g de [(tert-butoxycarbonylamino-2 phényl) acétyl]-2 diphényl-7,7 perhydroisoindolone-4-(3aRS,7aRS) par 15 cm³ d'une solution 5,7 N d'acide chlorhydrique dans le dioxanne sec à 20°C pendant 4 heures. Le mélange réactionnel est concentré à sec sous pression réduite (2,7 kPa) et le résidu purifié par dissolution dans 20 cm³ d'acétonitrile et précipitation par 30 cm³ d'oxyde d'isopropyle. Le solide est essoré, lavé par de l'oxyde d'isopropyle et séché. On obtient 1,1 g de chlorhydrate d'[(amino-2 phényl) acétyl]-2 diphényl-7,7 perhydroisoindolone-4-(3aRS,7aRS), sous la forme d'un solide légèrement rosé.

Spectre RMN du proton :
A température ambiante, on observe le mélange des deux rotamères.
2,1 et 2,27 (2Mt, respectivement 1 H chacun, -CH₂- en 5 ou 6) ; 2,65 à 3,35 (Mt, 4H, -CH₂- en 6 ou 5 et -CH₂- en 1) ; 3,4 à 3,75 (Mt, 1H du -CH₂- en 3 et 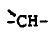 en 3a) ; 3,55 et 3,84 (2S, >N-CO-CH2-) 3,9 à 4,2 (Mt, ^{Î}CH- en 7a) ; 4,15 à 4,4 (Mt, 1 H du -CH₂- en 3 ; 7 à 7,7 (Mt, 14H, aromatiques).
   Spectre infra-rouge (KBr) bandes caractéristiques (cm⁻¹):
   3430, 3085, 3000-1900, 3055, 3025, 2965, 2880, 1715, 1630-1520, 1625, 1595, 1580, 1492, 1455, 755, 703

L'acide (tert-butoxycarbonylamino-2 phényl) acétique peut être obtenu de la manière suivante :
Une solution de 18,1 g d'acide (nitro-2 phényl) acétique dans 120 cm³ de solution normale de soude est hydrogénée en autoclave sous une pression de 5 bars en 2,5 heures à 20°C en présence de 1,5 g de palladium à 3 % sur noir de carbone. La solution du sel de sodium de l'acide (amino-2 phényl) acétique ainsi obtenu est refroidie à +5_{°}C puis traitée par une solution 26,16 g de dicarbonate de ditert-butyle dans 100 cm³ de tétrahydrofuranne puis par 80 cm³ de solution normale de soude. Le mélange réactionnel est agité à 20°C pendant 80 heures, concentré partiellement sous pression réduite (2,7 kPa), dilué par 200 cm³ d'eau et lavé 3 fois par 200 cm³ d'éther éthylique. La phase aqueuse est acidifiée à pH 3 par addition d'acide chlorhydrique 4 N et extraite par de l'acétate d'éthyle (2 fois 200 cm³). Les phases organiques réunies sont lavées par 150 cm³ d'eau (2 fois), séchées sur sulfate de magnésium, filtrées et concentrées à sec sous pression réduite (2,7 kPa). On obtient 25 g d'acide (tert-butoxycarbonylamino-2 phényl) acétique sous la forme d'un solide blanc crème.

### EXEMPLE DE REFERENCE 11

A une solution de 1,85 g d'acide (N-tert-butoxycarbonyl N-méthyl amino-2 phényl) acétique dans 30 cm³ de dichlorométhane sec, refroidie à +5_{°}C, on ajoute 1,13 g de N,N'-carbonyldiimidazole. On agite 30 minutes à +5_{°}C puis on ajoute une solution de 2,29 g de chlorhydrate de diphényl-7,7 perhydroisoindolone-4-(3aRS,7aRS) et de 1,9 cm³ de triéthylamine dans 20 cm³ de dichlorométhane. Le mélange réactionnel est agité à 20°C pendant heures, puis lavé par 200 cm³ d'eau (2 fois), séché sur sulfate de magnésium, filtré et concentré à sec sous pression réduite (2,7 kPa). Le résidu est chromatographié sur une colonne de gel de silice (0,2-0,063 mm, diamètre 4 cm, hauteur 50 cm) en éluant sous une pression de 0,7 bar d'azote par un mélange de cyclohexane et d'acétate d'éthyle (30/70 en volumes) et en recueillant des fractions de 125 cm³. Les fractions 22 à 34 sont réunies et concentrées à sec sous pression réduite (2,7 kPa) pour donner 2,8 g de [(N-tert-butoxycarbonyl N-méthyl amino-2 phényl) acétyl]-2 diphényl-7,7 perhydroisoindolone-4-(3aRS,7aRS) sous la forme d'une meringue blanche.

On traite 2,8 g de [(N-tert-butoxycarbonyl N-méthyl amino-2 phényl) acétyl]-2 diphényl-7,7 perhydroisoindolone-4-(3aRS,7aRS) par 30 cm³ d'une solution 5,7 N d'acide chlorhydrique dans le dioxanne sec à 20°C pendant 4 heures. On ajoute 100 cm³ d'oxyde d'isopropyle, le solide est essoré, lavé par de l'oxyde d'isopropyle et séché. On obtient 2,1 g de chlorhydrate de [(méthylamino-2 phényl) acétyl]-2 diphényl-7,7 perhydroisoindolone-4-(3aRS,7aRS), sous la forme d'un solide blanc. Spectre RMN du proton :
A température ambiante, on observe le mélange des deux rotamères.
2,1 à 2,27 (2Mt, respectivement 1 H chacun, -CH₂- en 5 ou 6) ; 2,65 à 3,35 (Mt, -CH₂- en 6 ou 5 et -CH₂- en 1) ; 2,82 et 2,87 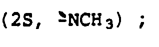 3,4 à 3,75 (Mt, 1 H du -CH₂- en 3 et 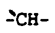 en 3a) ; 3,55 et 3,85 (2S, 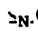 CO-CH₂-) ; 3,9 à 4,2 (Mt, 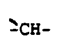 en 7a) ; 4,15 à 4,45 (Mt, 1 H du -CH₂- en 6 ou 5) ; 7 à 7,7 (Mt, 14H, aromatiques).

Spectre infra-rouge (KBr) bandes caractéristiques (cm⁻¹):
3600-3300, 3100-3000, 3000-2850, 3100-2200, 1712, 1640- 1610, 1595, 1495, 1475-1410, 1445, 755, 702. L'acide (N-tert-butoxycarbonyl N-méthyl amino-2 phényl) acétique peut être préparé de la manière suivante : Une solution de 3,5 g de (N-tert-butoxycarbonyl N-méthyl amino-2 phényl) acétate de méthyle dans 50 cm³ d'éthanol est traitée par 15 cm³ de solution de soude normale à 80°C pendant4 heures. Le mélange réactionnel est concentré sous pression réduite (2,7 kPa). Le résidu est repris par 100 cm³ d'eau, et la solution acidifiée à pH 1 par action d'acide chlorhydrique 4N est extraite par 100 cm³ d'acétate d'éthyle (2 fois).Les phases organiques réunies sont séchées sur sulfate de magnésium, filtrées et concentrées à sec sous pression réduite (2,7 kPa) pour donner 2,85 g d'acide (N-tert-butoxycarbonyl N-méthyl amino-2 phényl) acétique sous la forme d'un solide blanc.

Le (N-tertbutoxycarbonyl N-méthyl amino-2 phényl) acétate de méthyle peut être préparé de la manière suivante :
Une solution de 5 g d'acide (tert-butoxycarbonylamino-2 phényl) acétique dans 50 cm³ de diméthylformamide sec est ajoutée à une suspension de 1,2 g d'hydrure de sodium (dispersion à 80 % dans l'huile) dans 20 cm³ de diméthylformamide sec. Le mélange réactionnel est chauffé à 80°C pendant 2 heures, refroidi à 20°C. On ajoute 2,51 cm³ d'iodure de méthyle et agite à 20°C pendant 16 heures. On dilue par 200 cm³ d'eau et extrait par de l'acétate d'éthyle (2 fois 200 cm³). Les phases organiques réunies sont lavées par 100 cm³ d'eau, séchées sur sulfate de magnésium, filtrées et concentrées à sec sous pression réduite (2,7 kPa). Le résidu est chromatographié sur une colonne de gel de silice (0,2-0,063 mm, diamètre 4 cm, hauteur 48 cm) en éluant sous une pression de 0,7 bar d'azote par un mélange de cyclohexane et d'acétate d'éthyle (90/10 en volumes) et en recuei liant des fractions de 125 cm³. Les fractions 9 à 17 sont réunies et concentrées à sec sous pression réduite (2,7 kPa) pour donner 3,5 g de (N-tert-butoxycarbonyl N-méthyl amino-2 phényl) acétate de méthyle sous la forme d'une huile jaune.

### EXEMPLE DE REFERENCE 12

A une solution de 1,1 g d'acide diméthylamino-2 phénylacétique, dans 30 cm³ de dichlorométhane sec, refroidie à +5_{°}C, on ajoute 1 g de N,N'-carbonyldiimidazole. On agite 30 minutes à +5_{°}C puis on ajoute une solution de 2,03 g de chlorhydrate de diphényl-7,7 perhydroisoindolone-4-(3aRS,7aRS) et de 1,68 cm³ de triéthylamine dans 20 cm³ de dichlorométhane. Le mélange réactionnel est agité à 20°C pendant 24 heures, puis lavé par 200 cm³ d'eau (3 fois), séché sur sulfate de magnésium, filtré et concentré à sec sous pression réduite (2,7 kPa). Le résidu est chromatographié sur une colonne de gel de silice (0,2-0,063 mm, diamètre 3 cm, hauteur 25 cm) en éluant sous une pression de 0,7 bar d'azote, par un mélange de cyclohexane et d'acétate d'éthyle (40/60 en volumes) et en recueillant des fractions de 125 cm³. Les fractions 8 à 26 sont réunies et concentrées à sec sous pression réduite (2,7 kPa). Le résidu est cristallisé dans 40 cm³ d'oxyde d'isopropyle. Les cristaux sont essorés, lavés à l'oxyde d'isopropyle et séchés. On obtient 0,9 g de diphényl-7,7 [(di méthylamino-2 phényl)-2 acétyl]-2 perhydroisoindolone-4-(3aRS,7aRS) sous la forme de cristaux blancs, fondant à 150°C.

L'acide diméthylamino-2 phénylacétique est préparé selon la méthode de D-U. Lee, K.K. Mayeret W. Wie- grebe (Arch. Pharm. (Weinheim), 321, 303 (1988)).

### EXEMPLE DE REFERENCE 13

A une solution de 2,68 g d'acide diméthylamino-2 phénylacétique, dans 50 cm³ de dichlorométhane sec, refroidie à +5_{°}C, on ajoute 2,43 g de N,N'-carbonyldiimidazole. On agite 90 minutes à +5_{°}C puis on ajoute une solution de 4,9 g de chlorhydrate de diphényl-7,7 perhydroisoindolone-4-(3aR,7aR) et de 4,2 cm³ de triéthylamine dans 50 cm³ de dichlorométhane. Le mélange réactionnel est agité à 20°C pendant 16 heures, puis lavé par 100 cm³ d'eau (2 fois), séché sur sulfate de magnésium, filtré et concentré à sec sous pression réduite (2,7 kPa). Le résidu est chromatographié sur une colonne de gel de silice (0,2-0,063 mm, diamètre 5 cm, hauteur 50 cm) en éluant sous une pression de 0,7 bar d'azote, par un mélange de cyclohexane et d'acétate d'éthyle (40/60 en volumes) et en recueillant des fractions de 125 cm³. Les fractions 5 à 20 sont réunies et concentrées à sec sous pression réduite (2,7 kPa). Le résidu est cristallisé dans un mélange de 40 cm³ d'acétonitrile et 200 cm³ d'oxyde d'isopropyle. Les cristaux sont essorés, lavés à l'oxyde d'isopropyle et séchés. On obtient 2,58 g de diphényl-7,7 [(diméthylamino-2 phényl)-2 acétyl]-2 perhydroisoindolone-4-(3aR,7aR) sous la forme de cristaux blancs, fondant à 190°C ;
[a]D20= - 242° (c=1,18, chloroforme)

### EXEMPLE DE REFERENCE 14

A une solution de 0,62 g d'acide phényl-2 propionique-(S) dans 30 cm³ de dichlorométhane sec, refroidie à +5_{°}C, on ajoute 0,66 g de N,N'-carbonyldiimidazole. On agite 40 minutes à +5_{°}C puis on ajoute une solution de 1,35 g de chlorhydrate de diphényl-7,7 perhydroisoindolone-4-(3aR,7aR) et de 0,57 cm³ de triéthylamine dans 40 cm³ de dichlorométhane. Le mélange réactionnel est agité à 20°C pendant 16 heures, puis lavé par 50 cm³ d'eau distillée (2 fois), séché sur sulfate de magnésium, filtré et concentré à sec sous pression réduite (2,7 kPa). Le résidu est chromatographié sur une colonne de gel de silice (0,2-0,063 mm, diamètre 1,8 cm, hauteur 15 cm) en éluant par de l'acétate d'éthyle et en recueillant des fractions de 15 cm³. La première fraction est concentrée à sec sous pression réduite (2,7 kPa). On obtient 1 g de diphényl-7,7 [phényl-2 propionyl-(S)]-2 perhydroisoindolone-4-(3aR,7aR) sous la forme d'une meringue blanche;
[α]_{D}²⁰= -231⁰ (c=1, méthanol).

Spectre RMN du proton :
A température ambiante, on observe le mélange des deux rotamères.
1,16 et 1,26 (2D, 3H en totalité, -CH₃) ; 1,95 à 2,3 (Mt, 2H, -CH₂- en 5 ou 6) ; 2,65 à 2,9 (Mt, 4H, -CH₂- en 6 ou 5 et -CH₂- en 1) ; 3,05 à 3,35 (Mt, 2H, 1 H du -CH₂- en 3 et ^{E}CH- en 3a) ; 3,4 et 3,8 à 4 (Mt, 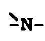 CO-CH-et 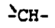 en 7a) ; 4,2 à 4,4 (Mt, 1 H, 1 H du -CH₂- en 3) ; 6,9 à 7,6 (Mt, 15H, aromatiques).
Spectre infra-rouge (KBr) bandes caractéristiques (cm⁻¹):
3600-3300, 3100-3000, 3000-2870, 1715, 1640, 1600, 1580, 1495, 1455, 1445, 1420, 1370, 755, 700.

### EXEMPLE DE REFERENCE 15

Aune solution de 0,75 g d'acide [méthoxy-2 phényl]-2 propionique-(S), à 84 % de pureté optique, préparé selon T. Matsumoto et collaborateurs: Bull. Chem. Soc. Jpn., 58, 340 (1985) dans 15 cm³ de diméthylformamide sec, on ajoute 0,59 g d'hydroxy-1 benzotriazole, puis refroidit la solution à 0°C. On ajoute 0,91 g de N,N'- dicyclohexylcarbodiimide, agite 1 heure à cette température puis on ajoute une solution de 1,44 g de chlorhydrate de diphényl-7,7 perhydroisoindolone-4-(3aR,7aR) et de 0,76 cm³ de N N'-diisopropyléthylamine dans 10 cm³ de diméthylformamide. Le mélange réactionnel est agité à 20°C pendant 16 heures, dilué par 100 cm³ d'acétate d'éthyle et concentré à sec sous pression réduite (2,7 kPa) après filtration du précipité. Le résidu est chromatographié sur une colonne de gel de silice (0,2-0,063 mm, diamètre 3 cm, hauteur40 cm) en éluant sous une pression de 0,7 bar d'azote, par un mélange de cyclohexane et d'acétate d'éthyle (50/50 en volumes) et en recueillant des fractions de 125 cm³. Les fractions 4 à 7 sont réunies et concentrées à sec sous pression réduite (2,7 kPa). Le résidu est purifié par dissolution dans 60 cm³ d'oxyde d'isopropyle bouillant additionné de 30 cm³ d'hexane. La solution refroidie est filtrée et le filtrat concentré à sec sous pression réduite (2,7 kPa) pour donner 1,2 g de diphényl-7,7 [(méthoxy-2 phényl)-2 propionyl-(S)]-2 perhydroisoindolone-4-(3aR,7aR) sous la forme d'une meringue blanche contenant 10 % de diphényl-7,7 [(méthoxy-2 phényl)-2 propionyl-(R)]-2 perhydroisoindolone-4-(3aR,7aR) ;
[α]_{D}²⁰= - 1810 (c=0,81, chloroforme).

Spectre RMN du proton :
A température ambiante, on observe le mélange des deux rotamères de chacun des deux diastéréoisomères. Les deux diastéréoisomères étant dans les proportions 90/10.
1,10 et 1,20 (2 Mt, 3H en totalité, - CH₃) ; 1,9 à 2,4 (Mt, 2H, -CH₂-en 5 ou 6) ; 2,55 à 2,95 (Mt, -CH₂- en 1 et - CH₂- en 6 ou 5) ; 2,95 à 3,4 (Mt, 1 H du -CH₂- en 3 et CH- en 3a) ; 3,20-3,32-3,50 et 3,83 4S, -OCH₃) ; 3,65 à 4,3 (Mt, CH- en 7a, N-CO-CH-, 1 H du -CH₂- en 3) ; 6,7 à 7,65 (Mt, 14H, aromatiques).
Spectre infra-rouge (KBr), bandes caractéristiques (cm-¹) :
3430, 3100-3000, 3000-2800, 1715, 1640, 1595, 1585, 1490, 1460, 1445, 1420, 1365, 1240, 1030, 755, 703.

### EXEMPLE DE REFERENCE 16

A une solution de 1 g d'acide [diméthylamino-2 phényl]-2 propionique-(RS) dans 30 cm³ de dichlorométhane refroidie à 5°C, on ajoute 0,85 g de N,N'-carbonyldiimidazole puis agite 30 minutes à cette température. On ajoute une solution de 1,7 g de chlorhydrate de diphényl-7,7 perhydroisoindolone-4-(3aR,7aR) et de 1,4 cm³ de triéthylamine dans 30 cm³ de dichlorométhane. Le mélange réactionnel est agité à 20°C pendant 16 heures, lavé 2 fois par 100 cm³ d'eau, séché sur sulfate de magnésium et concentré à sec sous pression réduite (2,7 kPa). Le résidu est chromatographié sur une colonne de gel de silice (0,2-0,063 mm, diamètre 3 cm, hauteur 50 cm) en éluant sous une pression de 0,5 bar d'azote, par de l'acétate d'éthyle et en recueillant des fractions de 125 cm³. Les fractions 4 à 6 sont réunies et concentrées à sec sous pression réduite (2,7 kPa) pour donner 1,4 g de [(diméthylamino-2 phényl)-2 propionyl-(RS)]-2 diphényl-7,7 perhydroisoindolone-4(3aR,7aR) sous la forme d'une meringue blanche.

Spectre RMN du proton :
A température ordinaire, on observe le mélange des deux rotamères de chacun des deux diastéréoisomères. 1,15 à 1,35 (Mt, 3H, -CH₃) ; 1,9 à 2,4 (Mt, -CH₂- en 5 ou 6); 2,1 - 2,19 - 2,62 - 2,64 (45, -N(CH3)2); 2,55 à 3,4 (Mt, -CH₂- en 6 ou 5, -CH₂- en 1, -CH- en 3a et 1 H du -CH₂- en 3) ; 3,5 à 4,5 (Mt, -N-CO-CH-, 1 H du -CH₂- en 3 et -CH en 7a) ; 7 à 7,7 (Mt, 15H, aromatiques).
Spectre infra-rouge (KBr), bandes caractéristiques (cm-¹) :
3600-3300, 3100-3000, 3000-2780, 1715, 1640, 1595, 1580, 1490, 1460, 1445, 1410, 750, 702.

Une solution de 1,8 g d'acide [diméthylamino-2 phényl]-2 acétique dans 10 cm³ de tétrahydrofuranne sec est ajoutée à 10°C à une solution de diisopropylamidure de lithium (préparée par action de 2,6 cm³ de solution 1,6 M de butyllithium dans l'hexane sur une solution de 2,8 g de diisopropylamine dans 30 cm³ de tétrahydrofuranne sec à 10°C). Le mélange réactionnel est agité 30 minutes à 20°C puis 30 minutes à 35°C. Après refroidissement à 20°C on ajoute 0,63 cm³ d'iodure de méthyle et chauffe 1 heure à 35°C. On refroidit, dilue par 20 cm³ d'eau et 100 cm³ d'acétate d'éthyle. La phase aqueuse est lavée par 100 cm³ d'acétate d'éthyle, acidifiée à pH 5 par de l'acide chlorhydrique et extraite 2 fois par 100 cm³ d'acétate d'éthyle. Les phases organiques sont lavées à l'eau, séchées sur sulfate de magnésium et concentrées à sec sous pression réduite (2,7 kPa) pour donner 1 g d'acide [diméthylamino-2 phényl]-2 propionique-(RS) sous la forme d'une huile jaune.

### EXEMPLE DE REFERENCE 17

A une solution de 2 g de chlorhydrate de diphényl-7,7 hexahydro-2,3,3a,4,7,7a 1 H-isoindolone-4 et de 1,7 cm³ de triéthylamine dans 20 cm³ de dichlorométhane sec, refroidie à +5°C, on ajoute 0,82 cm³ de chlorure de phénylacétyle. Le mélange réactionnel est agité 1 heure à +5_{°}C et 1 heure à température ambiante ; il est lavé par 20 cm³ d'eau distillée (2 fois), séché sur sulfate de magnésium, filtré et concentré à sec sous pression réduite (2,7 kPa). Le résidu est cristallisé dans 15 cm³ d'acétonitrile. Les cristaux sont essorés, lavés par 10 cm³ d'oxyde d'isopropyle, séchés, puis recristallisés dans 20 cm³ d'acétonitrile. Les cristaux obtenus sont essorés et séchés. On obtient 2,7 g de diphényl-7,7 (phénylacétyl)-2 hexahydro-2,3,3a,4,7,7a 1 H-isoindolone-4-(3aRS,7aRS), fondant à 188°C.

### EXEMPLE DE REFERENCE 18

Une suspension de 1,5 g de chlorhydrate de bis(fluoro-3 phényl)-7,7 perhydroisoindolone-4 dans 30 cm³ de dichlorométhane refroidie à +4_{°}C est traitée par 1,15 cm³ de triéthylamine puis par 0,63 g de chlorure de phénylacétyle. Le mélange réactionnel est agité 5 heures à 25°C, puis lavé 3 fois par 100 cm³ d'eau. La phase organique est séchée sur sulfate de magnésium, filtrée et concentrée à sec sous pression réduite (2,7 kPa). Le résidu est chromatographié sur une colonne de gel de silice (granulomètrie 0,04-0,063 mm, diamètre 2,3 cm, hauteur25 cm) en éluant sous une pression de 0,5 bard'azote par un mélange de cyclohexane et d'acétate d'éthyle (55/45 en volumes) pour donner 1,21 g de meringue qui est cristallisée par addition de 10 cm³ d'oxyde d'isopropyle. Les cristaux sont essorés, lavés par de l'oxyde d'isopropyle et séchés. On obtient 0,76 g de bis(fluoro-3 phényl)-7,7 (phénylacétyl)-2 perhydroisoindolone-4-(3aRS,7aRS). P.F. = 108°C.

### EXEMPLE DE REFERENCE 19

Une solution de 0,46 g d'acide (méthoxy-2 phényl)acétique dans 15 cm³ de dichlorométhane sec est refroidie à 0°C puis traitée par 0,45 g de N,N'-carbonyldiimidazole et agitée pendant 1 heure à 0°C. On ajoute goutte à goutte une solution de 1 g de chlorhydrate de bis(fluoro-3 phényl)-7,7 perhydroisoindolone-4 et 0,76 cm³ de triéthylamine dans 20 cm³ de dichlorométhane. Le mélange réactionnel est agité 3 heures à 25°C, puis lavé 2 fois par 50 cm³ d'eau et par 50 cm³ de solution saturée de chlorure de sodium. La phase organique est séchée sur sulfate de magnésium, filtrée et concentrée à sec sous pression réduite (2,7 kPa). Le résidu est chromatographié sur une colonne de gel de silice (granulomètrie 0,04-0,063 mm, diamètre 2,2 cm, hauteur 23 cm) en éluant sous une pression de 0,5 bar d'azote par un mélange de cyclohexane et d'acétate d'éthyle (70/30 en volumes) et en recueillant des fractions de 15 cm³. Les fractions 4 à 9 sont réunies et concentrées à sec sous pression réduite (2,7 kPa) et le produit obtenu est recristallisé dans de l'acétonitrile. Les cristaux sont essorés, lavés par de l'oxyde d'isopropyle et séchés. On obtient 0,76 g de bis(fluoro-3 phényl)-7,7 ((méthoxy-2 phényl)acétyl)-2 perhydroisoindolone-4-(3aRS,7aRS). P.F. = 194°C.

### EXEMPLE DE REFERENCE 20

Une suspension de 0,9 g de (méthoxy-2 phényl)acétamide dans 3 cm³ de dichlorométhane sec est traitée par 1,14 g de tétrafluoroborate de triéthyloxonium et la solution obtenue agitée pendant 20 heures à 25°C. Après refroidissement à 0°C on ajoute au milieu réactionnel une solution de 1,5 g de chlorhydrate de bis(fluoro-3 phényl)-7,7 perhydroisoindolone-4 et 1,4 cm³ de triéthylamine dans 9 cm³ de dichlorométhane. Le mélange réactionnel est agité 30 minutes à 25°C, puis chauffé au reflux pendant 5 heures et enfin agité encore 16 heures à 25°C. On ajoute 50 cm³ de solution saturée de carbonate de potassium, agite, filtre et lave la phase organique 2 fois par 50 cm³ d'eau. Après séchage sur sulfate de magnésium, filtration et concentration à sec sous pression réduite (2,7 kPa), le résidu est chromatographié sur une colonne d'alumine (diamètre 2,6 cm, hauteur 24 cm) en éluant sous une pression de 0,5 bar d'azote par un mélange de dichloro-1,2 éthane et de méthanol (95/5 en volumes) et en recueillant des fractions de 15 cm³. Les fractions 7 à 25 sont réunies et concentrées à sec sous pression réduite (2,7 kPa) pour donner 0,54 g de bis(fluoro-3 phényl)-7,7 [imino-1 (méthoxy-2 phényl)-2 éthyl]-2 perhydroisoindolone-4-(3aRS,7aRS) sous la forme d'une meringue jaune pâle.

Spectre RMN du proton (CDCI₃):
2,20 et 2,45 (2m, 2H, -CH₂- en 5) ; 2,8 (m, 2H, -CH₂- en 6) ; 3,08 (m, 2H, -CH₂- en 1) ; 3,23 (m, 1 H, H en 3a) ; 3,53 (dd,J=11 et 6,5, 1 H, 1 H du -CH₂- en 3) ; 3,6 (s, 2H, -CH₂-Ar) ; 3,8 (m, 1 H, H en 7a) ; 3,8 (s, 3H, OCH3) ; 4,43 (d, J=11, 1 H, 1 H du -CH₂- en 3) ; 6,8 à 7,5 (m, 14H aromatiques).
Spectre infrarouge (bandes caractéristiques en cm-'): 3425, 3100-3000, 3000-2850, 2835, 1715, 1592, 1610, 1595,1460,1250,1030,780,755,695.

### EXEMPLE DE REFERENCE 21

A une solution de 0,65 g d'acide (diméthylamino-2 phényl)acétique, dans 20 cm³ de dichlorométhane sec refroidie à +4_{°}C, on ajoute 0,59 g de N,N'-carbonyle diimidazole. Le mélange est agité 90 minutes à 25 °C puis on ajoute goutte à goutte une solution de 1,3 g de chlorhydrate de bis(fluoro-3 phényl)-7,7 perhydroisoindolone-4 et de 1,02 cm³ de triéthylamine dans 25 cm³ de dichlorométhane sec. Le mélange réactionnel est agité 16 heures à 25°C et lavé 2 fois par 250 cm³ d'eau et par 250 cm³ de solution saturée de chlorure de sodium. La phase organique est séchée sur sulfate de magnésium et concentrée à sec sous pression réduite (2,7 kPa). Le résidu est chromatographié sur une colonne de gel de silice (granulomètrie 0,04-0,063 mm, diamètre 2,3 cm, hauteur 23 cm) en éluant sous une pression de 0,5 bar d'azote par un mélange de cyclohexane et d'acétate d'éthyle (55/45) et en recueillant des fractions de 15 cm³. Les fractions 6 à 18 sont réunies et concentrées à sec sous pression réduite (2,7 kPa). Le résidu est concrèté par de l'oxyde d'isopropyle pour donner 0,6 g de bis(fluoro-3 phényl)-7,7 (diméthylamino-2 phényl)acétyl-2 perhydroisoindolone-4-(3aRS,7aRS) dont on prépare le chlorhydrate par dissolution dans 1 cm³ d'acétate d'éthyle et addition d'une solution 3N d'acide chlorhydrique dans l'oxyde d'isopropyle. Le précipité est essoré, lavé par de l'oxyde d'iso- propyle et séché. On obtient 0,48 g de chlorhydrate de bis(fluoro-3 phényl)-7,7 (diméthylamino-2 phényl)acétyl-2 perhydroisoindolone-4-(3aRS,7aRS) sous la forme d'un solide blanc.

Spectre RMN du proton (DMSO-dₛ/AcOD 90/10):
A température ordinaire on observe le mélange des deux rotamères. 2 à 2,32 (m, 2H, -CH₂- en 5) ; 2,37 et 2,6 (2s, 3H chacun, -N(CH₃)₂) ; 2,65 à 3 (m, 4H, -CH₂- en 6 et -CH₂- en 1) ; 3,15 à 3,3 (m, 1 H, H en 3a) ; 3,35 et 3,47 (2m, 1 H, 1 H en 3) ; 3,35 et 3,5 (2d, J=15, ArCH₂CO d'un rotamère) ; 3,67 (s, ArCH₂CO de l'autre rotamère) ; 4 (m, 1 H, H en 7a) ; 4,2 et 4,25 (2m, J=11, 1 H , 1 H en 3) ; 6,9 à 7,6 (m, 12H, aromatiques)
Spectre infrarouge (bandes caractéristiques en cm-'): 3500-3150, 3100-3000, 3000-2850, 1712, 1650, 1615, 1595, 1580, 1495, 1445, 1535, 755, 700.

### EXEMPLE DE REFERENCE 22

A une solution de 0,49 g d'acide (diméthylamino-2 phényl)acétique, dans 20 cm³ de dichlorométhane sec refroidie à +4_{°}C, on ajoute 0,44 g de N,N'-carbonyle diimidazole. Le mélange est agité 1 heure à 25 °C puis on ajoute goutte à goutte une solution de 1 g de chlorhydrate de bis(fluoro-2 phényl)-7,7 perhydroisoindolone-4 et de 0,76 cm³ de triéthylamine dans 25 cm³ de dichlorométhane sec. Le mélange réactionnel est agité 20 heures à 25°C et lavé 2 fois par 100 cm³ d'eau et par 100 cm³ de solution saturée de chlorure de sodium. La phase organique est séchée sur sulfate de magnésium et concentrée à sec sous pression réduite (2,7 kPa). Le résidu est chromatographié sur une colonne de gel de silice (granulomètrie 0,04-0,063 mm, diamètre 2 cm, hauteur 23 cm) en éluant sous une pression de 0,5 bar d'azote par un mélange de cyclohexane et d'acétate d'éthyle (50/50) et en recueillant des fractions de 10 cm³. Les fractions 14 à 36 sont réunies et concentrées à secsous pression réduite (2,7 kPa) pour donner 1 g de bis(fluoro-2 phényl)-7,7 (diméthylamino-2 phényl)acétyl-2 perhydroisoindolone-4-(3aRS,7aRS) dont on prépare le chlorhydrate pardissolution dans 2 cm³ d'acétate d'éthyle et addition d'une solution 3N d'acide chlorhydrique dans l'oxyde d'isopropyle. Le précipité est essoré, lavé par de l'oxyde d'isopropyle et séché. On obtient 0,87 g de chlorhydrate de bis(fluoro-2 phényl)-7,7 (diméthylamino-2 phényl)acétyl-2 perhydroisoindolone-4-(3aRS,7aRS) sous la forme d'un solide blanc. Spectre RMN du proton (DMSO-d₆/AcOD 90/10) ; à température ambiante on observe le mélange des deux rotamères: 2,1 à 2,35 (m, 2H, -CH₂- en 5) ; 2,8 à 3,4 (m, 10H, -CH₂- en 1 et en 6 , N(CH₃)₂) ; 3,7 et 3,5 (2 dd larges, 1 H, H en 3a) ; 3,8 (dd large, 1 H, 1 H en 3) ; 4,05 (s large, 2H, -CH₂CO) ; 4,1 (m large, 1 H, H en 7a) ; 4,2 et 4,45 (d, 1 H, 1 H en 3) ; 7 à 8 (m, 12H aromatiques).

### EXEMPLE DE REFERENCE 23

A une solution de 1,06 g de chlorhydrate de bis(chloro-3 phényl)-7,7 perhydroisoindolone-4 dans 20 cm³ de dichlorométhane refroidie à +4_{°}C on ajoute 0,45 cm³ de triéthylamine puis 0,49 g de chlorure de phénylacétyle. Le mélange réactionnel est agité 2 heures à 25°C, puis lavé 3 fois par 30 cm³ d'eau et 3 par par 30 cm³ de solution saturée de chlorure de sodium. La phase organique est séchée sur sulfate de magnésium, filtrée et concentrée à sec sous pression réduite (2,7 kPa). Le résidu est chromatographié sur une colonne de gel de silice (granulomètrie 0,04-0,063 mm, diamètre 2,2 cm, hauteur 23 cm) en éluant sous une pression de 0,5 bar d'azote par un mélange de cyclohexane et d'acétate d'éthyle (55/45) et en recueillant des fractions de 15 cm³. Les fractions 7 à 18 sont concentrées à sec sous pression réduite (2,7 kPa) et le résidu est cristallisé dans de l'acétonitrile. Les cristaux sont essorés, lavés par de l'oxyde d'isopropyle et séchés. On obtient 0,21 g de bis(chloro-3 phényl)-7,7 (phénylacétyl)-2 perhydroisoindolone-4-(3aRS,7aRS). P.F. = 160°C.

### EXEMPLE DE REFERENCE 24

Une suspension de 1,5 g de chlorhydrate de bis(tolyl-3)-7,7 perhydroisoindolone-4-(3aRS,7aRS) dans 30 cm³ de dichlorométhane refroidie à +4_{°}C est traitée par 1,15 cm³ de triéthylamine puis par 0,63 g de chlorure de phénylacétyle. Le mélange réactionnel est agité 5 heures à 25°C, puis lavé 3 fois par 100 cm³ d'eau. La phase organique est séchée sur sulfate de magnésium, filtrée et concentrée à sec sous pression réduite (2,7 kPa). Le résidu est cristallisé 2 fois dans l'acétonitrile pour donner 0,36 g de bis(tolyl-3)-7,7 (phénylacétyl)-2 perhydroisoindolone-4- (3aRS,7aRS). P.F. = 207°C.

## Revendications (Revendications pour l'(les) Etat(s) contractant(s) suivant(s) : suivants : AT, BE, CH, DE, DK, FR, GB, IT, LI, LU, NL, SE)

1. Un dérivé de l'isoindolone de formule générale : 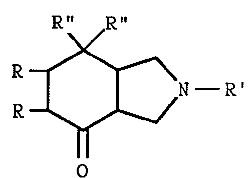 caractérisé en ce que les radicaux R représentent des atomes d'hydrogène ou forment ensemble une liaison, le symbole R' est choisi parmi l'atome d'hydrogène, un radical allyle ou un radical de structure : 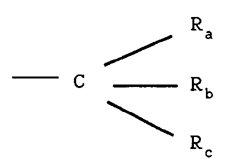 dans laquelle Rₐ et R_{b} sont des atomes d'hydrogène ou des radicaux phényle éventuellement substitués (par un atome d'halogène, un radical alcoyle, alcoyloxy ou nitro), et Rₑ est défini comme Rₐ et R_{b} ou représente un radical alcoyle ou alcoyloxyalcoyle, l'un au moins de Rₐ, R_{b} et Rₑ étant un radical phényle substitué ou non, étant entendu que les radicaux et portions alcoyle cités ci-dessus sont droits ou ramifiés et contiennent 1 à 4 atomes de carbone, et les symboles R" sont identiques et représentent des radicaux phényle pouvant être substitués par un atome d'halogène ou un radical méthyle en position ortho ou méta, sous forme (3aR,7aR) ou sous forme d'un mélange des formes (3aRS,7aRS), ainsi que ses sels d'addition avec les acides.

2. Un dérivé de l'isoindolone selon la revendication 1, caractérisé en ce que les radicaux R sont des atomes d'hydrogène ou forment ensemble une liaison, le symbole R' est un atome d'hydrogène ou un radical benzyle et les symboles R" sont des radicaux phényle éventuellement substitués en position ortho ou méta par des atomes de fluor ou de chlore, ou par un radical méthyle.

3. Un dérivé de l'isoindolone selon la revendication 1, caractérisé en ce qu'il s'agit de la diphényl-7,7 perhydroisoindolone-4 sous ses formes (3aR,7aR) ou (3aRS,7aRS), ainsi que ses sels d'addition avec les acides.

4. Un dérivé de l'isoindolone selon la revendication 1, caractérisé en ce qu'il s'agit de la bis(fluoro-3 phényl)-7,7 perhydroisoindolone-4 sous ses formes (3aR,7aR) ou (3aRS,7aRS), ainsi que ses sels d'addition avec les acides.

5. Un dérivé de l'isoindolone selon la revendication 1, caractérisé en ce qu'il s'agit de la bis(fluoro-2 phényl)-7,7 perhydroisoindolone-4 sous ses formes (3aR,7aR) ou (3aRS,7aRS), ainsi que ses sels d'addition avec les acides.

6. Un dérivé de l'isoindolone selon la revendication 1, caractérisé en ce qu'il s'agit de la bis(chloro-3 phényl)-7,7 perhydroisoindolone-4 sous ses formes (3aR,7aR) ou (3aRS,7aRS), ainsi que ses sels d'addition avec les acides.

7. Un dérivé de l'isoindolone selon la revendication 1, caractérisé en ce qu'il s'agit de la bis(tolyl-3)-7,7 perhydroisoindolone-4 sous ses formes (3aR, 7aR) ou (3aRS,7aRS), ainsi que ses sels d'addition avec les acides.

8. Procédé de préparation d'un dérivé de l'isoindolone selon la revendication 1 ou 2, caractérisé en ce que l'on fait agir un dérivé silylé de formule générale : 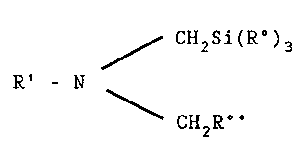 dans laquelle R' est un radical facilement éliminable défini dans la revendication 1 ou dans la revendication 2, (R°)₃ représente des radicaux alcoyle ou des radicaux alcoyle et phényle et R°° représente un radical alcoyloxy, cyano ou phénylthio sur un dérivé de la cyclohexènone de formule générale : 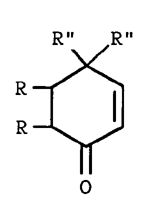 dans laquelle R et R" sont définis comme dans la revendication 1, puis éventuellement élimine le radical R' facilement éliminable lorsque l'on veut obtenir une isoindolone pour laquelle R' est un atome d'hydrogène, sépare éventuellement les isomères et transforme éventuellement le produit obtenu en un sel.

9. Procédé de préparation d'un dérivé de l'isoindolone selon la revendication 1 ou la revendication 2, caractérisé en ce que l'on fait agir une oxazolidinone de formule générale : 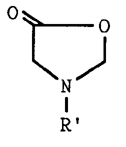 dans laquelle R' est le groupement facilement éliminable tel que défini précédemment dans la revendication 1 ou 2, sur un dérivé de la cyclohexènone de formule générale : 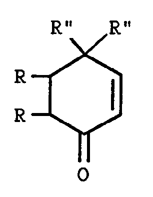 dans laquelle R et R" sont définis comme dans la revendication 1, puis le cas échéant, lorsque l'on veut obtenir un dérivé de l'oxazolidinone pour lequel R' est un atome d'hydrogène, élimine le radical R' facilement éliminable, sépare éventuellement les isomères et transforme éventuellement le produit obtenu en un sel.

10. Procédé de préparation d'un dérivé de l'isoindolone selon la revendication 1 ou 2, pour lequel R est un atome d'hydrogène et R' est autre que trityle, caractérisé en ce que l'on met en oeuvre une réaction de Mannich à partir d'un produit de formule générale : 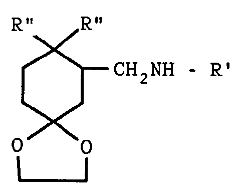 dans laquelle R' est défini comme ci-dessus et R" est défini comme dans la revendication 1, puis sépare éventuellement les isomères et transforme éventuellement le produit obtenu en un sel.

11. Procédé de préparation d'un dérivé de l'isoindolone selon la revendication 1 pour lequel R et R' sont des atomes d'hydrogène, caractérisé en ce que l'on effectue l'hydrogénation catalytique d'une formyl-2 nitrométhyl-3 cyclohexanone de formule : 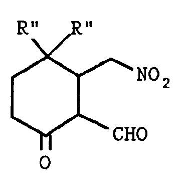 dans laquelle R" est défini comme dans la revendication 1, puis sépare éventuellement les isomères et transforme éventuellement le produit obtenu en un sel.

12. Utilisation d'un produit selon la revendication 1, pour la préparation d'un dérivé de l'isoindolone de formule générale : 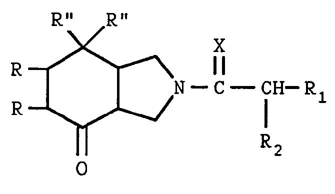 dans laquelle
- les radicaux R sont identiques et représentent des atomes d'hydrogène ou forment ensemble une liaison,
- les radicaux R" sont définis comme dans la revendication 1,
- le symbole X représente un atome d'oxygène, de soufre ou un radical N-R₃ pour lequel R₃ est un atome d'hydrogène, un radical alcoyle contenant 1 à 12 atomes de carbone, éventuellement substitué [par un ou plusieurs radicaux carboxy, dialcoylamino, acylamino, carbamoyle, alcoylcarbamoyle, dialcoylcarbamoyle, alcoyloxycarbonyle (les portions alcoyle de ces radicaux pouvant porter un substituant dialcoylamino ou phényle), ou par des radicaux phényle, phényle substitué par (halogène, alcoyle, alcoyloxy ou dialcoylamino), naphtyle, thiényle, furyle, pyridyle ou imidazolyle] ou un radical dialcoylamino,
- le symbole R₁ représente un radical phényle éventuellement substitué par un ou plusieurs atomes d'halogène ou radicaux hydroxy, alcoyle pouvant être éventuellement substitués (par des atomes d'halogène ou des radicaux amino, alcoylamino ou dialcoylamino), alcoyloxy ou alcoylthio pouvant être éventuellement substitués (par des radicaux hydroxy ou dialcoylamino dont les parties alcoyle peuvent former avec l'atome d'azote auquel elles sont rattachées, un hétérocycle à 5 à 6 chaînons pouvant contenir un autre hétéroatome choisi parmi l'oxygène, le soufre ou l'azote éventuellement substitué par un radical alcoyle), ou substitué par des radicaux amino, alcoylamino, dialcoylamino dont les parties alcoyle peuvent former avec l'atome d'azote auquel elles sont rattachées, un hétérocycle tel que défini ci-dessus, ou représente un radical cyclohexadiènyle, naphtyle ou hétérocyclyle mono ou polycyclique, saturé ou insaturé contenant 5 à 9 atomes de carbone et un ou plusieurs hétéroatomes choisis parmi l'oxygène, l'azote ou le soufre et
- le symbole R₂ représente un atome d'hydrogène ou d'halogène ou un radical hydroxy, alcoyle, ami- noalcoyle, alcoylaminoalcoyle, dialcoylaminoalcoyle, alcoyloxy, alcoylthio, alcyloxy, carboxy, alcoyloxycarbonyle, dialcoylaminoalcoyloxycarbonyle, benzyloxycarbonyle, amino, acylamino ou alcoy- loxycarbonylamino.

## Revendications (Revendications pour l'(les) Etat(s) contractant(s) suivant(s) : ES, GR)

1. Procédé de préparation d'un dérivé de l'isoindolone de formule générale : 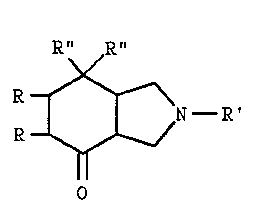 dans laquelle les radicaux R représentent des atomes d'hydrogène ou forment ensemble une liaison, le symbole R' représente un atome d'hydrogène ou un radical allyle ou un radical de structure : 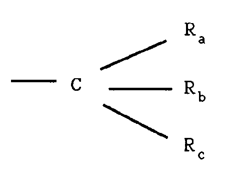 dans laquelle Rₐ et R_{b} sont des atomes d'hydrogène ou des radicaux phényle éventuellement substitués (par un atome d'halogène, un radical alcoyle, alcoyloxy ou nitro), et Rₑ est défini comme Rₐ et R_{b} ou représente un radical alcoyle ou alcoyloxyalcoyle, l'un au moins de Rₐ, R_{b} et Rₑ étant un radical phényle substitué ou non, étant entendu que les radicaux et portions alcoyle cités ci-dessus sont droits ou ramifiés et contiennent 1 à 4 atomes de carbone et les symboles R" sont identiques et représentent des radicaux phényle pouvant être substitués par un atome d'halogène ou un radical méthyle en position ortho ou méta, sous forme (3aR,7aR) ou sous forme d'un mélange des formes (3aRS,7aRS), ainsi que ses sels d'addition avec les acides, caractérisé en ce que l'on fait agir un dérivé silylé de formule générale : 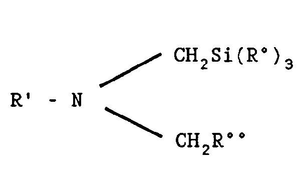 dans laquelle R' est un radical facilement éliminable défini ci-dessus, (R°)₃ représente des radicaux alcoyle ou des radicaux alcoyle et phényle et R°° représente un radical alcoyloxy, cyano ou phénylthio sur un dérivé de la cyclohexènone de formule générale : 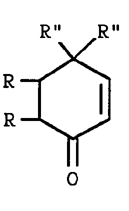 dans laquelle R et R" sont définis comme ci-dessus, puis éventuellement élimine le radical R' facilement éliminable lorsque l'on veut obtenir une isoindolone pour laquelle R' est un atome d'hydrogène, ou bien caractérisé en ce que l'on fait agir une oxazolidinone de formule générale : 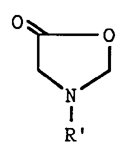 dans laquelle R' est le groupement facilement éliminable tel que défini précédemment, sur un dérivé de la cyclohexènone de formule générale : 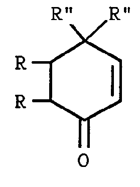 dans laquelle R et R" sont définis comme ci-dessus, puis le cas échéant, lorsque l'on veut obtenir un dérivé de l'oxazolidinone pour lequel R' est un atome d'hydrogène, élimine le radical R' facilement éliminable, ou bien
pour préparer un dérivé de l'isoindolone pour lequel R est un atome d'hydrogène et R' est autre que trityle, caractérisé en ce que l'on met en oeuvre une réaction de Mannich à partir d'un produit de formule générale : 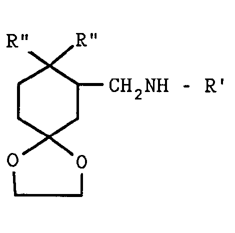 dans laquelle R" est défini comme précédemment et R' est défini comme ci-dessus, ou bien
pour préparer un dérivé de l'isoindolone pour lequel R et R' sont des atomes d'hydrogène, caractérisé en ce que l'on effectue l'hydrogénation catalytique d'une formyl-2 nitrométhyl-3 cyclohexanone de formule : 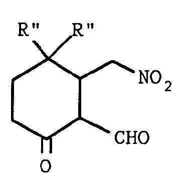 dans laquelle R" est défini comme précédemment, puis sépare éventuellement les isomères et transforme éventuellement le produit obtenu en un sel.

## Patentansprüche (Patentansprüche für folgende(n) Vertragsstaat(en) : AT, BE, CH, DE, DK, FR, GB, IT, LI, LU, NL, SE)

1. Isoindolon-Derivat der allgemeinen Formel: 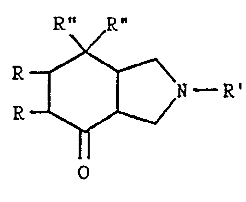 dadurch gekennzeichnet, daß die Reste R Wasserstoffatome darstellen oder zusammen eine Bindung bilden, das Symbol R' ausgewählt ist aus dem Wasserstoffatom, einem Allylrest oder einem Rest der Struktur: 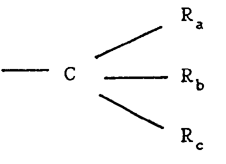 in der Rₐ und R_{b} Wasserstoffatome oder gegebenenfalls (durch ein Halogenatom, einen Alkyl-, Alkyloxy-oder Nitrorest) substituierte Phenylreste sind und Rₑ wie Rₐ und R_{b} definiert ist oder einen Alkyl- oder Alkyloxyalkylrest darstellt, wobei mindestens eines von Rₐ, R_{b} und Rₑ ein substituierter oder unsubstituierter Phenylrest ist, wobei verstanden wird, daß die oben aufgeführten Alkylreste und -teile geradkettig oder Verzweigt sind und 1 bis 4 Kohlenstoffatome enthalten, und die Symbole R" identisch sind und Phenylreste, die durch ein Halogenatom oder einen Methylrest in ortho- oder meta-Stellung substituiert sein können, darstellen, in der Form (3aR, 7aR) oder in Form einer Mischung der Formen (3aRS, 7aRS), sowie dessen Additionssalze mit Säuren.

2. Isoindolon-Derivat nach Anspruch 1, dadurch gekennzeichnet, daß die Reste R Wasserstoffatome sind oder zusammen eine Bindung bilden, das Symbol R' ein Wasserstoffatom oder ein Benzylrest ist und die Symbole R" gegebenenfalls in ortho- oder meta-Stellung durch Fluor- oder Chloratome oder durch einen Methylrest substituierte Phenylreste sind.

3. Isoindolon-Derivat nach Anspruch 1, dadurch gekennzeichnet, daß es sich um 7,7-Diphenylperhydroisoindol-4-on in seinen Formen (3aR, 7aR) oder (3aRS, 7aRS) sowie dessen Additionssalze mit Säuren handelt.

4. Isoindolon-Derivat nach Anspruch 1, dadurch gekennzeichnet, daß es sich um 7,7-Bis(3-fluorphenyl)per- hydroisoindol-4-on in seinen Formen (3aR, 7aR) oder (3aRS, 7aRS) sowie um dessen Additionssalze mit Säuren handelt.

5. Isoindolon-Derivat nach Anspruch 1, dadurch gekennzeichnet, daß es sich um 7,7-Bis(2-fluorphenyl)per- hydroisoindol-4-on in seinen Formen (3aR, 7aR) oder (3aRS, 7aRS) sowie um dessen Additionssalze mit Säuren handelt.

6. Isoindolon-Derivat nach Anspruch 1, dadurch gekennzeichnet, daß es sich um 7,7-Bis(3-chlorphenyl)per- hydroisoindol-4-on in seinen Formen (3aR, 7aR) oder (3aRS, 7aRS) sowie um dessen Additionssalze mit Säuren handelt.

7. Isoindolon-Derivat nach Anspruch 1, dadurch gekennzeichnet, daß es sich um 7,7-Bis(3-tolyl)perhydroi- soindol-4-on in seinen Formen (3aR, 7aR) oder (3aRS, 7aRS) sowie um dessen Additionssalze mit Säuren handelt.

8. Verfahren zur Herstellung eines Isoindolon-Derivats nach Anspruch 1 oder 2, dadurch gekennzeichnet, daß man ein Silyl-Derivat der allgemeinen Formel: 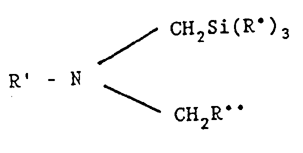 in der R' ein in Anspruch 1 oder in Anspruch 2 definierter leicht eliminierbarer Rest ist, (R0)3Alkylreste oder Alkyl- und Phenylreste darstellt und R°° einen Alkyloxy-, Cyano- oder Phenylthiorest darstellt, auf ein Cyclohexenon-Derivat der allgemeinen Formel: 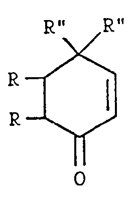 in der R und R" wie in Anspruch 1 definiert sind, einwirken läßt, dann gegebenenfalls den leicht eliminierbaren Rest R' eliminiert, wenn man ein Isoindolon erhalten will, bei dem R' ein Wasserstoffatom ist, gegebenenfalls die Isomeren auftrennt und gegebenenfalls das erhaltene Produkt in ein Salz überführt.

9. Verfahren zur Herstellung eines Isoindolon-Derivats nach Anspruch 1 oder nach Anspruch 2, dadurch gekennzeichnet, daß man ein Oxazolidinon der allgemeinen Formel: 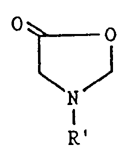 in der R' die leicht eliminierbare Gruppe wie vorstehend in Anspruch 1 oder 2 definiert ist, auf ein Cyclohexenon-Derivat der allgemeinen Formel: 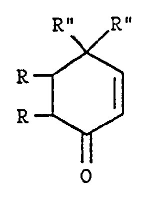 in der Rund R" wie in Anspruch 1 definiert sind, einwirken läßt, dann gegebenenfalls, wenn man ein Oxazolidinon-Derivat erhalten will, bei dem R' ein Wasserstoffatom ist, den leicht eliminierbaren Rest R' eliminiert, gegebenenfalls die Isomeren auftrennt und gegebenenfalls das erhaltene Produkt in ein Salz überführt.

10. Verfahren zur Herstellung eines Isoindolon-Derivats nach Anspruch 1 oder 2, bei dem Rein Wasserstoffatom ist und R' von Trityl verschieden ist, dadurch gekennzeichnet, daß man eine Mannich-Reaktion ausgehend von einem Produkt der allgemeinen Formel: 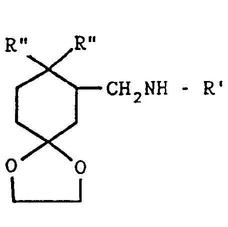 in der R' wie oben definiert ist und R" wie in Anspruch 1 definiert ist, durchführt, dann gegebenenfalls die Isomeren auftrennt und das erhaltene Produkt gegebenenfalls in ein Salz überführt.

11. Verfahren zur Herstellung eines Isoindolon-Derivats nach Anspruch 1, bei dem R und R' Wasserstoffatome sind, dadurch gekennzeichnet, daß man eine katalytische Hydrierung eines 2-Formyl-3-nitrome- thylcyclohexanons der Formel: 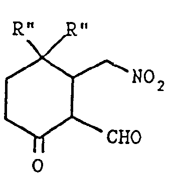 in der R" wie in Anspruch 1 definiert ist, durchführt, dann gegebenenfalls die Isomeren auftrennt und gegebenenfalls das erhaltene Produkt in ein Salz überführt.

12. Verwendung eines Produkts nach Anspruch 1 für die Herstellung eines Isoindolon-Derivats der allgemeinen Formel: 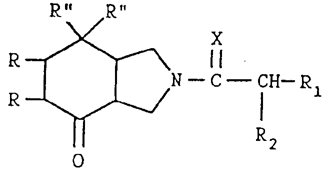 in der
- die Reste R identisch sind und für Wasserstoffatome stehen oder zusammen eine Bindung bilden,
- die Reste R" wie in Anspruch 1 definiert sind,
- das Symbol X für ein Sauerstoff-, Schwefelatom oder einen Rest N-R₃ steht, bei dem R₃ ein Wasserstoffatom, ein Alkylrest mit 1 bis 12 Kohlenstoffatomen, der gegebenenfalls substituiert ist [durch einen oder mehrere Carboxy-, Dialkylamino-, Acylamino-, Carbamoyl-, Alkylcarbamoyl-, Dialkylcarbamoyl-, Alkyloxycarbonylreste (wobei die Alkylteile dieser Reste einen Dialkylamino- oder Phenylsubstituenten tragen können), oder durch Phenyl-, (durch Halogen, Alkyl, Alkyloxy oder Dialkylamino) substituierte Phenyl-, Naphthyl-, Thienyl-, Furyl-, Pyridyl- oder Imidazolylreste], oder ein Dialkylaminorest ist,
- das Symbol R₁ für einen Phenylrest steht, gegebenenfalls substituiert durch ein oder mehrere Halogenatome oder einen oder mehrere Hydroxy-, Alkylreste, die gegebenenfalls substituiert sein können (durch Halogenatome oder Amino-, Alkylamino- oder Dialkylaminoreste) , Alkyloxy- oder Alkylthioreste, die gegebenenfalls substituiert sein können (durch Hydroxy- oder Dialkylaminoreste, deren Alkylteile mit dem Stickstoffatom, an das sie gebunden sind, einen Heterocyclus mit 5 bis 6 Gliedern bilden können, der ein anderes Heteroatom, ausgewählt aus Sauerstoff, Schwefel oder Stickstoff, gegebenenfalls durch einen Alkylrest substituiert, enthalten kann), oder substituiert durch Amino-, Alkylamino-, Dialkylaminoreste, deren Alkylteile mit dem Stickstoffatom, an das sie gebunden sind, einen Heterocyclus wie oben definiert bilden können, oder für einen Cyclohexadienyl-, Naphthylrest oder einen mono- oder polycyclischen Heterocyclus, gesättigt oder ungesättigt, mit 5 bis 9 Kohlenstoffatomen und einem oder mehreren Heteroatomen, ausgewählt aus Sauerstoff, Stickstoff oder Schwefel, steht und
- das Symbol R₂ für ein Wasserstoff- oder Halogenatom oder einen Hydroxy-, Alkyl-, Aminoalkyl-, Alkylaminoalkyl-, Dialkylaminoalkyl-, Alkyloxy-, Alkylthio-, Acyloxy-, Carboxy-, Alkyloxycarbonyl-, Dialkylaminoalkyloxycarbonyl-, Benzyloxycarbonyl-, Amino-, Acylamino- oder Alkyloxycarbonylaminorest steht.

## Patentansprüche (Patentansprüche für folgende(n) Vertragsstaat(en) : ES, GR)

1. Verfahren zur Herstellung eines Isoindolon-Derivats der allgemeinen Formel: 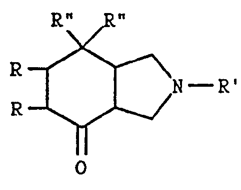 in der die Reste R Wasserstoffatome darstellen oder zusammen eine Bindung bilden, das Symbol R' ein Wasserstoffatom oder ein Allylrest oder einen Rest der Struktur: 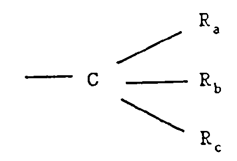 darstellt, in der Rₐ und R_{b} Wasserstoffatome oder gegebenenfalls (durch ein Halogenatom, einen Alkyl-, Alkyloxy- oder Nitrorest) substituierte Phenylreste sind und R_{c} wie Rₐ und R_{b} definiert ist oder einen Alkyl-oder Alkyloxyalkylrest darstellt, wobei mindestens eines von Rₐ, R_{b} und R_{c} ein substituierter oder unsubstituierter Phenylrest ist, wobei verstanden wird, daß die oben aufgeführten Alkylreste und -teile geradkettig oder verzweigt sind und 1 bis 4 Kohlenstoffatome enthalten, und die Symbole R" identisch sind und Phenylreste, die durch ein Halogenatom oder einen Methylrest in ortho- oder meta-Stellung substituiert sein können, darstellen, in der Form (3aR, 7aR) oder in Form einer Mischung der Formen (3aRS, 7aRS), sowie von dessen Additionssalzen mit Säuren, dadurch gekennzeichnet, daß man ein Silyl-Derivat der allgemeinen Formel: 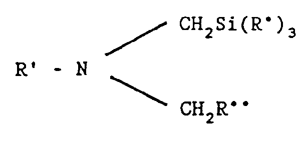 in der R' ein oben definierter leicht eliminierbarer Rest ist, (R°)₃ Alkylreste oder Alkyl- und Phenylreste darstellt und R°° einen Alkyloxy-, Cyano- oder Phenylthiorest darstellt, auf ein Cyclohexenon-Derivat der allgemeinen Formel: 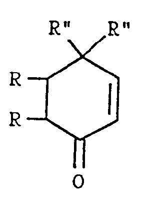 in der R und R" wie oben definiert sind, einwirken läßt, dann gegebenenfalls den leicht eliminierbaren Rest R' eliminiert, wenn man ein Isoindolon erhalten will, bei dem R' ein Wasserstoffatom ist, oder auch dadurch gekennzeichnet, daß man ein Oxazolidinon der allgemeinen Formel: 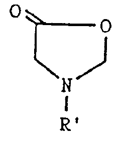 in der R' die leicht eliminierbare Gruppe wie vorstehend definiert ist, auf ein Cyclohexenon-Derivat der allgemeinen Formel: 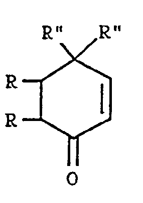 in der Rund R" wie oben definiert sind, einwirken läßt, dann gegebenenfalls, wenn man ein Oxazolidinon-Derivat erhalten will, bei dem R'ein Wasserstoffatom ist, den leicht eli minierbaren Rest R' eliminiert, oder auch, um ein Isoindolon-Derivat herzustellen, bei dem R ein Wasserstoffatom ist und R' von Trityl verschieden ist, dadurch gekennzeichnet, daß man eine Mannich-Reaktion ausgehend von einem Produkt der allgemeinen Formel: 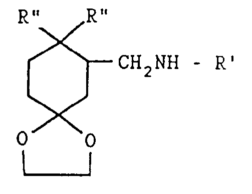 in der R" wie vorstehend definiert ist und R' wie oben definiert ist, durchführt, oder auch,
um ein Isoindolon-Derivat herzustellen, bei dem R und R' Wasserstoffatome sind, dadurch gekennzeichnet, daß man eine katalytische Hydrierung eines 2-Formyl-3-nitromethylcyclohexanons der Formel: 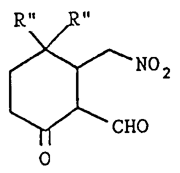 in der R" wie vorstehend definiert ist, durchführt, dann gegebenenfalls die Isomeren auftrennt und gegebenenfalls das erhaltene Produkt in ein Salz überführt.

## Claims (Claims for the following Contracting State(s) : AT, BE, CH, DE, DK, FR, GB, IT, LI, LU, NL, SE)

1. An isoindolone derivative of general formula: 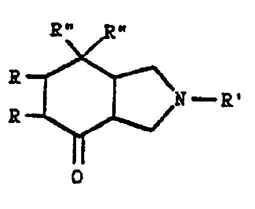 characterized in that the radicals R represent hydrogen atoms or together form a bond, the symbol R' is chosen from the hydrogen atom, an allyl radical or a radical of structure: 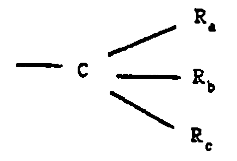 in which Rₐ and R_{b} are hydrogen atoms or phenyl radicals which are optionally substituted (with a halogen atom or an alkyl, alkyloxy or nitro radical), and Rₑ is defined as Rₐ and R_{b} or represents an alkyl or alky- loxyalkyl radical, at least one of Rₐ, R_{b} and Rₑ being a substituted or unsubstituted phenyl radical, it being understood that the alkyl radicals and portions mentioned above are straight or branched and contain 1 to 4 carbon atoms, and the symbols R" are identical and represent phenyl radicals which may be substituted with a halogen atom or a methyl radical in the ortho or meta position, in the (3aR,7aR) form, or in the form of a mixture of the (3aRS,7aRS) forms, as well as its addition salts with acids.

2. An isoindolone derivative according to claim 1, characterized in that the radicals R are hydrogen atoms or together form a bond, the symbol R' is a hydrogen atom or a benzyl radical and the symbols R" are phenyl radicals which are optionally substituted in the ortho or meta position with fluorine or chlorine atoms, or with a methyl radical.

3. An isoindolone derivative according to claim 1, characterized in that it is 7,7-diphenylperhydro-4-iso-in- dolone in its (3aR,7aR) or (3aRS,7aRS) forms, as well as its addition salts with acids.

4. An isoindolone derivative according to claim 1, characterized in that it is 7,7-bis(3-fluorophenyl)- perhy- dro-4-isoindolone in its (3aR,7aR) or (3aRS,7aRS) forms, as well as its addition salts with acids.

5. An isoindolone derivative according to claim 1, characterized in that it is 7,7-bis(2-fluorophenyl)-perhydro-4-isoindolone in its (3aR,7aR) or (3aRS,7aRS) forms, as well as its addition salts with acids.

6. An isoindolone derivative according to claim 1, characterized in that it is 7,7-bis(3-chlorophenyl)-perhy- dro-4-isoindolone in its (3aR,7aR) or (3aRS,7aRS) forms, as well as its addition salts with acids.

7. An isoindolone derivative according to claim 1, characterized in that it is 7,7-bis(3-tolyl)perhydro-4-isoindolone in its (3aR,7aR) or (3aRS,7aRs) forms, as well as its addition salts with acids.

8. Process for the preparation of an isoindolone derivative according to claim 1 or 2, characterized in that a silylated derivative of general formula: 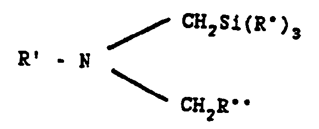 in which R' is an easily eliminated radical defined in claim 1 or claim 2, (R*)₃ represents alkyl radicals or alkyl and phenyl radicals and Roo represents an alkyloxy, cyano or phenylthio radical, is reacted with a cyclo-hexenone derivative of general formula: 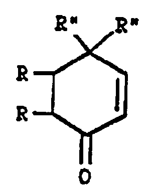 in which Rand R" are defined as in claim 1, then the easily eliminated radical R' is optionally eliminated when it is desired to obtain an isoindolone for which R' is a hydrogen atom, the isomers are optionally separated and the product obtained is optionally converted to a salt.

9. Process for the preparation of an isoindolone derivative according to claim 1 or claim 2, characterized in that an oxazolidinone of general formula: 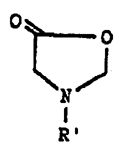 in which R' is the easily eliminated group as defined above in claim 1 or 2, is reacted with a cyclohexenone derivative of general formula: 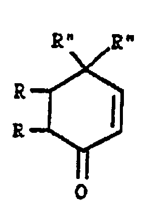 in which R and R" are defined as in claim 1, then, where appropriate, when it is desired to obtain an oxazolidinone derivative for which R' is a hydrogen atom, the easily eliminated radical R' is eliminated, the isomers are optionally separated and the product obtained is optionally converted to a salt.

10. Process for the preparation of an isoindolone derivative according to claim 1 or2 forwhich R is a hydrogen atom and R' is other than trityl, characterized in that a Mannich reaction is carried out, starting from a product of general formula: 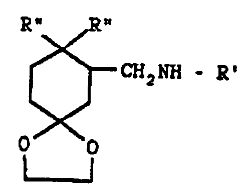 in which R' is defined as above and R" is defined as in claim 1, then the isomers are optionally separated and the product obtained is optionally converted to a salt.

11. Process for the preparation of an isoindolone derivative according to claim 1 for which Rand R' are hydrogen atoms, characterized in that a catalytic hydrogenation is carried out on a 2-formyl-3-nitromethyl- cyclohexanone of formula: 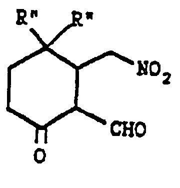 in which R" is defined as in claim 1, then the isomers are optionally separated and the product obtained is optionally converted to a salt.

12. Use of a product according to claim 1, for the preparation of an isoindolone derivative of general formula: 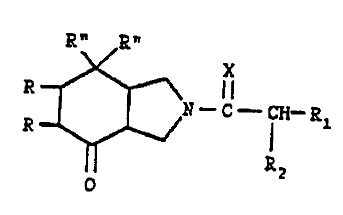 in which
- the radicals R are identical and represent hydrogen atoms or together form a bond,
- the radicals R" are defined as in claim 1,
- the symbol X represents an oxygen or sulphur atom, or a radical N-R₃ for which R₃ is a hydrogen atom or an alkyl radical containing 1 to 12 carbon atoms, optionally substituted [with one or more carboxyl, dialkylamino, acylamino, carbamoyl, alkylcarbamoyl, dialkylcarbamoyl, alkyloxycarbonyl (it being possible for the alkyl portions of these radicals to carry a dialkylamino or phenyl substituent) radicals, or with phenyl, phenyl substituted with (halogen, alkyl, alkyloxy or dialkylamino), naphthyl, thienyl, furyl, pyridyl or imidazolyl radicals], or a dialkylamino radical,
- the symbol R₁ represents a phenyl radical which is optionally substituted with one or more halogen atoms or hydroxyl radicals, alkyl radicals which may be optionally substituted (with halogen atoms or amino, alkylamino or dialkylamino radicals), alkyloxy or alkylthio radicals which may be optionally substituted (with hydroxyl radicals or dialkylamino radicals in which the alkyl parts can form, with the nitrogen atom to which they are attached, a 5- to 6-membered heterocycle which may contain another heteroatom chosen from oxygen, sulphur or nitrogen which is optionally substituted with an alkyl radical), or substituted with amino, alkylamino or dialkylamino radicals in which the alkyl parts may form, with the nitrogen atom to which they are attached, a heterocycle as defined above, or represents a cyclohexadienyl or naphthyl radical or a saturated or unsaturated mono- or polycyclic heterocyclic radical containing 5 to 9 carbon atoms and one or more hetero-atoms chosen from oxygen, nitrogen or sulphur and
- the symbol R₂ represents a hydrogen or halogen atom or a hydroxyl, alkyl, aminoalkyl, alkylaminoalkyl, dialkylaminoalkyl, alkyloxy, alkylthio, acyloxy, carboxyl, alkyloxycarbonyl, dialkylaminoalkyloxy- carbonyl, benzyloxycarbonyl, amino, acylamino or alkyloxycarbonylamino radical.

## Claims (Claims for the following Contracting State(s) : ES, GR)

1. Process for the preparation of an isoindolone derivative of general formula: 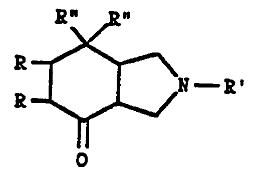 in which the radicals R represent hydrogen atoms or together form a bond, the symbol R' represents a hydrogen atom or an allyl radical or a radical of structure: 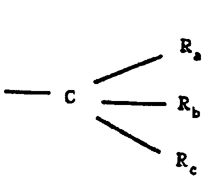 in which Rₐ and R_{b} are hydrogen atoms or phenyl radicals which are optionally substituted (with a halogen atom or an alkyl, alkyloxy or nitro radical), and Rₑ is defined as Rₐ and R_{b} or represents an alkyl or alky- loxyalkyl radical, at least one of Rₐ, R_{b} and Rₑ being a substituted or unsubstituted phenyl radical, it being understood that the alkyl radicals and portions mentioned above are straight or branched and contain 1 to 4 carbon atoms, and the symbols R" are identical and represent phenyl radicals which may be substituted with a halogen atom or a methyl radical in the ortho or meta position, in the (3aR,7aR) form, or in the form of a mixture of the (3aRS,7aRS) forms, as well as its addition salts with acids, characterized in that a silylated derivative of general formula: 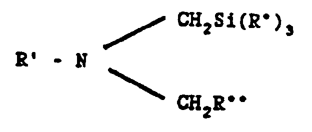 in which R' is an easily eliminated radical defined above, (R^{.})₃ represents alkyl radicals or alkyl and phenyl radicals and R^{..} represents an alkyloxy, cyano or phenylthio radical, is reacted with a cyclohexenone derivative of general formula: 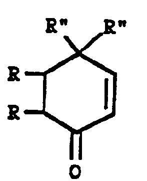 in which Rand R" are defined as above, then the easily eliminated radical R' is optionally eliminated when it is desired to obtain an isoindolone for which R' is a hydrogen atom, or else
characterized in that an oxazolidinone of general formula: 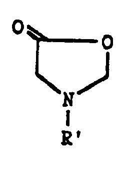 in which R' is the easily eliminated group as defined above, is reacted with a cyclohexenone derivative of general formula: 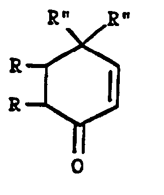 in which R and R" are defined as above, then, where appropriate, when it is desired to obtain an oxazolidinone derivative for which R' is a hydrogen atom, the easily eliminated radical R' is eliminated, or else in order to prepare an isoindolone derivative for which R is a hydrogen atom and R' is other than trityl, characterized in that a Mannich reaction is carried out, starting from a product of general formula: 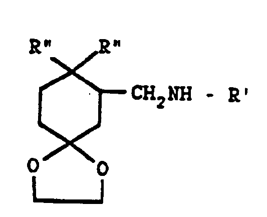 in which R" is defined as above and R' is defined as above, or else
in order to prepare an isoindolone derivative for which Rand R' are hydrogen atoms, characterized in that a catalytic hydrogenation is carried out on a 2-formyl-3-nitromethylcyclohexanone of formula: 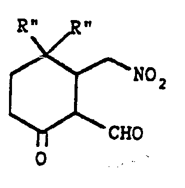 in which R" is defined as above, then the isomers are optionally separated and the product obtained is option- ally converted to a salt.
